(19) Europäisches Patentamt
European Patent Office
Office européen des brevets

(11) Publication number: **0 216 785**
**B1**

(12) # EUROPEAN PATENT SPECIFICATION

(45) Date of publication of patent specification: **30.01.91**

(21) Application number: **86900099.2**

(22) Date of filing: **29.11.85**

(86) International application number:
**PCT/EP85/00653**

(87) International publication number:
**WO 86/03488 19.06.86 Gazette 86/13**

(51) Int. Cl.[5]: **C 07 C 69/738,**
C 07 C 69/732, C 07 C 59/90,
C 07 C 59/56, C 07 C 59/54,
C 07 D 309/30,
A 61 K 31/365, A 61 K 31/22,
A 61 K 31/19

(54) **INDENE ANALOGS OF MEVALONOLACTONE AND DERIVATIVES THEREOF.**

(30) Priority: **04.12.84 US 677917**

(43) Date of publication of application:
**08.04.87 Bulletin 87/15**

(45) Publication of the grant of the patent:
**30.01.91 Bulletin 91/05**

(84) Designated Contracting States:
**AT BE CH DE FR GB IT LI LU NL SE**

(56) References cited:
**EP-A- 11 928 EP-A-0 114 027**
**EP-A- 113 881 EP-A-0 117 228**

**Journal of Medicinal Chemistry, vol.28, no. 43, March 1985, Columbus , Ohio, (US) G.E. STOKKER ET AL.:"3-hydroxy-3-methyl-glutaryal-coenzyme a reductase inhibitors. 1. Structural modification of 5-Substituted 3,5-dihydroxypenanoic adics and their lactone derivatives", pages 347-358**

**The file contains technical information submitted after the application was filed and not included in this specification**

(73) Proprietor: **SANDOZ AG**
**Lichtstrasse 35**
**CH-4002 Basel (CH)**
(84) **BE CH FR GB IT LI LU NL SE**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN Verwaltungsgesellschaft m.b.H.**
**Brunner Strasse 59**
**A-1235 Wien (AT)**
(84) **AT**

(72) Inventor: **KATHAWALA, Faizulla, G.**
**39 Woodland Avenue**
**Mountain Lakes, NJ 07946 (US)**
Inventor: **WATTANASIN, Sompong**
**30, Beech Street**
**Stanhope, NJ 078748 (US)**

**EP 0 216 785 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention concerns indene analogs of mevalonolactone and derivatives thereof, processes for their production, pharmaceutical compositions containing them and their use as pharmaceuticals in particular as hypolipoproteinemic and anti-atherosclerotic agents.

The invention is especially concerned with compounds of formula I

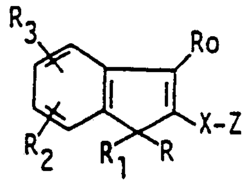

I

wherein

R is hydrogen or primary or secondary $C_{1-6}$alkyl,

$R_1$ is primary or secondary $C_{1-6}$alkyl or

R and $R_1$ together are $(CH_2)_m$ or (Z)—$CH_2$—$CH=CH$—$CH_2$— wherein m is 2, 3, 4, 5 or 6,

Ro is $C_{1-6}$alkyl, $C_{3-7}$cycloalkyl or ring A

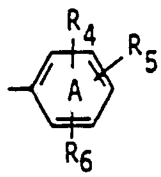

each of $R_2$ and $R_4$ is independently hydrogen, $C_{1-4}$alkyl, $C_{1-4}$alkoxy (except t-butoxy), trifluoromethyl, fluoro, chloro, phenoxy or benzyloxy,

each of $R_3$ and $R_5$ is independently hydrogen, $C_{1-3}$alkyl, $C_{1-3}$alkoxy, trifluoromethyl, fluoro, chloro, phenoxy or benzyloxy,

$R_6$ is hydrogen, $C_{1-2}$alkyl, $C_{1-2}$alkoxy, fluoro or chloro, with the proviso that there may only be one each of trifluoromethyl, phenoxy or benzyloxy on each of the phenyl and indene rings

X is —$(CH_2)_n$— or —$(CH_2)_qCH=CH(CH_2)_q$— wherein n is 1, 2 or 3 and both q's are 0 or one is 0 and theother is 1, and

Z is 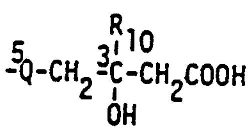

II

wherein

Q is 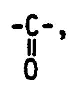, 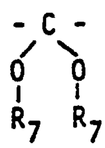 or 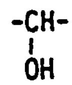

wherein each

$R_7$ is the same primary or secondary $C_{1-6}$alkyl or together they represnt —$(CH_2)_2$—, —$(CH_2)_3$—,

$R_{10}$ is hydrogen or $C_{1-3}$alkyl,

with the proviso that Q may be other than

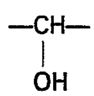

only when X is —CH=CH— or —$CH_2$—CH=CH— and/or $R_{10}$ is $C_{1-3}$alkyl, in free acid form, or in the form of an ester or -lactone thereof or in salt form as appropriate.

Suitable esters include physiologically acceptable esters e.g. physiologically hydrolysable and -acceptable esters.

By the term "physiologically-hydrolysable and -acceptable ester" is meant an ester of a compound in accordance with the invention in which the carboxyl moiety if present is esterified, and which is hydrolysable under physiological conditions to yield an alcohol which is itself physiologically acceptable, e.g. non-toxic at desired dosage levels. For the avoidance of doubt, throughout this application it is the

right hand side of the X radical that is attached to the Z group. Preferred such esters as Z can be represented together with the free acid by formula IIa

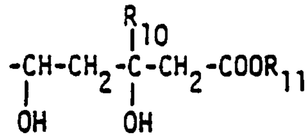

IIa

or formula IIC

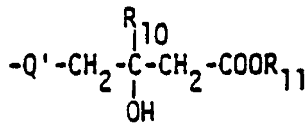

IIc

wherein $R_{11}$ is hydrogen, $C_{1-4}$alkyl or benzyl preferably hydrogen, $C_{1-3}$alkyl, n-butyl, i-butyl, t-butyl or benzyl,

Q' is 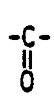 or 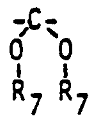

and $R_7$ and $R_{10}$ are as defined above with the further proviso that $R_{11}$ is other than hydrogen when

Q' is 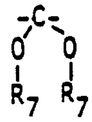

When IIa is in salt form $R_{11}$ represents a cation.

When Z is in lactone form it forms a δ-lactone of formula IIb

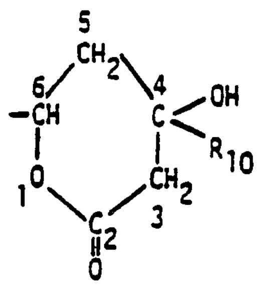

IIb

and references to "lactone" hereinafter refer to δ-lactones.

Salts of the compounds of the invention, e.g. of the compounds of formula I, include in particular their pharmaceutically acceptable salts. Such pharmaceutically acceptable salts iclude e.g. alkali metal salts such as the sodium and potassium salts and salts with ammonium.

References to compounds of formula I, II, IIa, IIb and IIc and sub-species thereof are intended to cover all forms unless otherwise stated.

Depending on the nature of $R_1$ and R the compounds of formula I may be divided into two main groups, namely, those wherein R is hydrogen or primary or secondary $C_{1-6}$alkyl (Group IA) and those wherein $R_1$ and R together represent $-(CH_2)_m-$ or (Z)$-CH_2-CH=CH-CH_2-$ (Group IB). These groups may be further divided depending on the nature of Z, namely when Q is

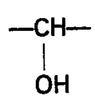

and the Z is in other than lactone form (sub-group "a"); when Z is a group of formula IIb (sub-group "b"); and when

Q is 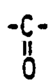 or 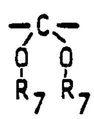

and Z is in other than lactone form (sub-group "c"). The resulting six groups are designated as IAa, IAb, IAc, IBa, IBb, IBc.

As is self-evident to those in the art, each compound of Groups IAa, IAb, IBa and IBb (and every subscope and species thereof) has two centres of asymmetry (the two carbon atoms bearing the hydroxy groups in the group of formula IIa and the carbon atom bearing the hydroxy group and the carbon atom having the free valence in the group of formula IIb and, therefore, there are four stereoisomeric forms (enantiomers) of each compound (two racemates or pairs of diastereoisomers), provided that R and $R_1$ are identical or taken together are $-(CH_2)_m-$ or $-(Z)-CH_2-CH=CH-CH_2-$ and that $R_{11}$ does not contain any centre of asymmetry. The four stereoisomers may be designated as the R,R, R,S, S,R and S,S enantiomers, all four stereoisomers being within the scope of this invention. When R and $R_1$ are different and/or $R_{11}$ contains one or more centres of asymmetry, there are eight or more stereoisomers. Since it is preferred that R and $R_1$ be identical or taken together are $-(CH_2)_m-$ or $(Z)-CH_2-CH=CH-CH_2-$ and that $R_{11}$ does not contain a centre of asymmetry and for reasons of simplicity any additional stereoisomers resulting from the presence of a centre of asymmetry in the 1-position of the indene ring and/or one or more centres of asymmetry in $R_{11}$ will usually be ignored, it being assumed that R and $R_1$ are identical or taken together are $-(CH_2)_m$ or $(Z)-CH_2-CH=CH-CH_2-$ and that $R_{11}$ is free of centres of asymmetry. As is also self-evident each compound of Groups IAc and IBc (and every subscope and species thereof) has one centre of asymmetry (the carbon atom bearing the hydroxy group in formula IIc and therefore there are two enantiomers of each compound, provided that R and $R_1$ are identical or taken together are $-(CH_2)_m$ or $(Z)-CH_2-CH=CH-CH_2-$ and that $R_{11}$ does not contain any centre of asymmetry. The two stereoisomers may be designated as the 3R and 3S isomers, both being within the scope of this invention. When R and $R_1$ are different and/or $R_{11}$ contains one or more centres of asymmetry, there are four or more stereoisomers. For the reasons set forth above, any additional stereoisomers resultings from the presence of a centre of asymmetry in the 1-position of the indene ring and/or one or more centres of asymmetry in $R_{11}$ will usually be ignored.

Ro preferably does not contain an asymmetric carbon atom and is preferably Ro′ where Ro′ is $C_{1-4}$alkyl not containing an asymmetric carbon atom or Ring A, more preferably Ro″, wherein Ro″ is ring A wherein $R_4$ is $R_4'$, $R_5$ is $R_5'$, and $R_6$ is $R_6'$ even more preferably Ro‴ where Ro‴ is ring A wherein $R_4$ is $R_4''$, $R_5$ is $R_5''$ and $R_6$ is $R_6''$ and most preferably Ro⁗ wherein Ro⁗ is ring A wherein $R_4$ is $R_4'''$, $R_5$ is $R_5'''$ an $R_6$ is $R_6'''$. In Ro⁗ $R_4'''$ is preferably $R_4''''$.

When R is hydrogen or primary or secondary $C_{1-6}$alkyl it is preferably hydrogen or primary or secondary $C_{1-6}$alkyl not containing an asymmetric carbon atom and is preferably R′, where R′ is hydrogen or primary or secondary $C_{1-4}$alkyl not containing an asymmetric carbon atom, more preferably R″ where R″ is hydrogen or $C_{1-2}$alkyl and most preferably $C_{1-2}$alkyl and $R_1$ is preferably primary or secondary $C_{1-6}$alkyl not containing any asymmetric carbon atom and is preferably $R_1'$, where $R_1'$ is primary or secondary $C_{1-4}$alkyl not containing an asymmetric carbon atom, more preferably $R_1''$, where $R_1''$ is $C_{1-3}$alkyl, and most preferably $C_{1-2}$alkyl.

Preferably, when R, R′, etc. is other than hydrogen, R, R′, etc., as the case may be, is identical to $R_1$, $R_1'$, etc., as the case may be.

When R and $R_1$ taken together are $-(CH_2)_m-$ or $(Z)-CH_2-CH=CH-CH_2-$, they are preferably $-(CH_2)_m-$, more preferably $-(CH_2)_{m'}-$, even more preferably $-(CH_2)_{m''}-$, and most preferably $-(CH_2)_{m'''}-$, especially $-(CH_2)_4-$, wherein m is as defined above, and m′, m″ and m‴ are as defined below.

$R_2$ is preferably hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy and is preferably $R_2'$, where $R_2'$ is hydrogen, $C_{1-3}$alkyl, methoxy, fluoro, chloro or benzyloxy, more preferably $R_2''$, where $R_2''$ is hydrogen or $C_{1-3}$alkyl, and most preferably hydrogen.

$R_3$ is preferably $R_3'$, where $R_3'$ is hydrogen or $C_{1-3}$alkyl, and more preferably hydrogen.

Preferably, not more than one of $R_2$ and $R_3$ is a member of the group consisting of *t*-butyl, trifluoromethyl, phenoxy and benzyloxy. More preferably, $R_2$ and $R_3$ are not *ortho* to each other unless at least one of them is a member of the group consisting of hydrogen, $C_{1-2}$alkyl, $C_{1-2}$alkoxy, fluoro and chloro.

$R_4$ is preferably hydrogen, $C_{1-3}$alkyl, n-butyl, i-butyl, t-butyl, $C_{1-3}$alkoxy, n-butoxy, i-butoxy, trifluoromethyl, fluoro, chloro, phenoxy or benzyloxy and is preferably $R_4'$, where $R_4'$ is hydrogen, $C_{1-3}$alkyl, trifluoromethyl, fluoro or chloro, more preferably $R_4''$, where $R_4''$ is hydrogen or $C_{1-2}$alkyl, and most preferably $R_4'''$, where $R_4'''$ is hydrogen or methyl, especially $R_4''''$, where $R_4''''$ is hydrogen or 3-methyl.

$R_5$ is preferably $R_5'$, where $R_5'$ is hydrogen, $C_{1-2}$alkyl, fluoro or chloro, more preferably $R_5''$ is hydrogen or fluoro, and most preferably $R_5'''$, where $R_5'''$ is hydrogen or 4-fluoro.

$R_6$ is preferably $R_6'$, where $R_6'$ is hydrogen or $C_{1-2}$alkyl, more preferably $R_6''$, where $R_6''$ is hydrogen or methyl, and most preferably $R_6'''$, where $R_6'''$ is hydrogen or 5-methyl.

Preferably, not more than one of $R_4$ and $R_5$ is a member of the group consisting of *t*-butyl, trifluoromethyl, phenoxy and benzyloxy. More preferably no two of $R_4$ ($R_4'$, $R_4''$, etc.), $R_5$ ($R_5'$, $R_5''$, etc.) and $R_6$ ($R_6'$, $R_6''$, etc.) are *ortho* to each other unless at least one member of each pair of substituents that are *ortho* to each other is a member of the group consisting of hydrogen, $C_{1-2}$alkyl, $C_{1-2}$alkoxy, fluoro and chloro.

The preferred $R_4$-bearing phenyl groups are phenyl, 4-fluorophenyl, 3,4- and 3,5-dimethylphenyl, 4-fluoro-3-methylphenyl and 3,5-dimethyl-4-fluorophenyl, with 4-fluorophenyl and 3,5-dimethylphenyl being more preferred.

Preferably each $R_7$ is $C_{1-3}$alkyl or both $R_7$'s taken together are $(CH_2)_2$ or $(CH_2)_3$; more preferably each $R_7$ is $C_{1-2}$alkyl or both $R_7$'s taken together are $(CH_2)_2$ or $(CH_2)_3$ and most preferably each $R_7$ is $C_{1-2}$alkyl.

$R_{10}$ is preferably $R_{10}'$, where $R_{10}'$ is hydrogen or methyl, and more preferably hydrogen.

$R_{11}$ is preferably $R_{11}'$, where $R_{11}'$ is hydrogen, $C_{1-3}$alkyl, n-butyl, i-butyl, t-butyl or benzyl especially $R_{11}''$ where $R_{11}''$ is hydrogen or $C_{1-3}$alkyl, more preferably $R_{11}'''$ which is hydrogen or $C_{1-2}$alkyl.

Compounds of formula I wherein Z is of formula II, IIa or IIc are most preferably in salt form. Preferred salt-forming cations are those free from centres of asymmetry especially e.g. sodium, potassium or ammonium, most preferably sodium. Such cations may also be di- or tri-valent and are balanced by 2 or 3 carboxylate containing anions. Any —CH=CH— containing bridge as X is preferably trans i.e. (E).

X is preferably X' which is $CH_2CH_2$ or —(E)—CH=CH—, more preferably —(E)—CH=CH—.

Z is preferably a group of formula IIa, IIb or IIc wherein $R_{10}$ is $R_{10}'$ (especially hydrogen) more preferably a group of formula IIa, IIb or IIc, wherein $R_{10}$ is hydrogen and $R_{11}$ is $R_{11}'$ or a cation, even more preferably a group of formula IIa or IIb wherein $R_{10}$ is hydrogen, and $R_{11}$ is $R_{11}''$ or a cation; and most preferably a group of formula IIa wherein $R_{10}$ is hydrogen, and $R_{11}$ is a cation, especially sodium.

m is preferably m', where m' is 2, 3, 4 or 5, more preferably m'', where m'' is 2, 3 or 4, and most preferably m''', where m''' is 2 or 4, especially 4.

n is preferably 2.

As between otherwise identical compounds of formula I, those wherein Z is other than lactone form are generally preferred over those wherein Z is a group of formula IIb, with those wherein Q is

$$\begin{array}{c} CH \\ | \\ OH \end{array}$$

being generally preferred over those wherein Q has another meaning.

Insofar as the compounds of Groups IAa and IBa and each of the subgroups thereof are concerned, the *erythro* isomers are preferred over the *threo* isomers, *erythro* and *threo* referring to the relative positions of the hydroxy groups in the 3- and 5-positions of the group of formula IIa.

Insofar as the compounds of Groups IAb and IBb and each of the subgroups thereof are concerned, the *trans* lactones are generally preferred over the *cis* lactones, *cis* and *trans* referring to the relative positions of $R_{10}$ and the hydrogen atom in the 6-position of the group of formula IIb.

The preferred stereoisomers of the compounds of formula I having only two centres of asymmetry wherein X is —CH=CH— or —$CH_2$—CH=CH— and Z is a group of formula IIa are the 3R,5S and 3R,5R isomers and the racemate of which each is a constituent, i.e. the 3R,5S—3S,5R (*erythro*) and 3R,5R—3S,5S (*threo*) racemates, with the 3R,5S isomer and the racemate of which it is a constituent being more preferred and the 3R,5S isomer being most preferred.

The preferred stereoisomers of the compounds of formula I having only two centres of asymmetry wherein X is —$(CH_2)_n$— or —CH=CH—$CH_2$— and Z is a group of formula IIa are the 3R,5R and 2R,5S isomers and the racemate of which each is a constituent, i.e. the 3R,5R—3S,5S (*erythro*) and 3R,5S—3S,5R (*threo*) racemates, with the 3R,5R isomer and the racemate of which it is a constituent being more preferred and the 3R,5R isomer being most preferred.

The preferred stereoisomers of the compounds of formula I having only two centres of asymmetry wherein X is —CH=CH— or —$CH_2$CH=CH— and Z is a group of formula IIb are then 4R,6S and 4R,6R isomers and the racemate of which each is a constituent, i.e. the 4R,6S—4S,6R (*trans* lactone) and 4R,6R—4S,6S (*cis* lactone) racemates, with the 4R,6S isomer and the racemate of which it is a constituent being more preferred and the 4R,6S isomer being most preferred.

The preferred stereoisomers of the compounds of formula I having only two centres of asymmetry wherein X is —$(CH_2)_n$— or —CH=CH—$CH_2$, and Z is a group of formula IIb are the 4R,6R and 4R,6S isomers and the racemate of which each is a constituent, i.e. the 4R,6R—4S,6S (*trans* lactone) and 4R,6S—4S,6R (*cis* lactone) racemates, with the 4R,6R isomer and the racemate of which it is a constituent being more preferred and the 4R,6S isomer being most preferred.

The preferences set forth in the preceding four paragraphs also apply to the compounds of Groups IAa, IAb, IBa, IBb having more than two centres of asymmetry and represent the preferred configurations of the indicated positions.

The preferred stereoisomers of the compounds of formula I having just one centre of asymmetry wherein Q is other than

$$\begin{array}{c} -CH- \\ | \\ OH \end{array}$$

are the 3R isomers and the racemate of which they are constituents i.e. the 3R—3S racemate with the 3R isomer being more preferred. These preferences also apply to the compounds of Groups IAc and IBc

having more than one centre of asymmetry and represent the preferred configuration of the indicated position.

Each of the preferences set forth above applies, not only to the compounds of formula I, but also to the compounds of Groups IAa, IAb, IAc, IBa, IBb and IBc as well as to every subgroup thereof set forth in the specification, e.g. Groups (i) et seq., unless otherwise indicated. When any preference contains a variable, the preferred significances of that variable apply to the preference in question, unless otherwise indicated.

Preferred groups of compounds of formula I include the compounds identified hereinafter by small Roman numerals between brackets:

(i) of Group IAa wherein Ro is Ro', R is R', $R_1$ is $R_1'$, $R_2$ is $R_2'$, $R_3$ is $R_3'$, $R_{10}$ is $R_{10}'$, $R_{11}$ is $R_{11}'$ and X is X'.

(ii) of (i) wherein Ro is R0", $R_{10}$ is hydrogen, $R_{11}$ is $R_{11}''$, and X is (E)—CH=CH—,

(iii) of (ii) wherein Ro is Ro''', R is R", $R_1$ is $R_1''$, $R_2$ is $R_2''$, $R_3$ is hydrogen, and $R_{11}$ is $R_{11}'''$, or especially a cation,

(iv) of (iii) wherein Ro is Ro''' wherein $R_4'''$ is $R_4''''$, R is $C_{1-2}$alkyl, $R_1$ is $C_{1-2}$ and $R_2$ is hydrogen,

(v) of (iv) wherein $R_{11}$ is a cation especially sodium, potassium or ammonium, especially sodium,

(vi) of Group IAb wherein Ro is Ro', R is R', $R_1$ is $R_1'$, $R_2$ is $R_2'$, $R_3$ is $R_3'$, $R_{10}$ is $R_{10}'$, and X is X',

(vii) of (vi) wherein Ro is Ro", $R_{10}$ is hydrogen, and X is (E)—CH=CH—,

(viii) of (vii) wherein Ro is Ro''', R is R", $R_1$ is $R_1''$, $R_2$ is $R_2''$ and $R_3$ is hydrogen,

(ix) of (viii) wherein Ro is Ro'''' wherein $R_4'''$ is $R_4''''$, R is $C_{1-2}$alkyl, $R_1$ is $C_{1-2}$alkyl and $R_2$ is hydrogen,

(x) of Group IBa wherein Ro is Ro', R and $R_1$ taken together are $—(CH_2)_m—$, $R_2$ is $R_2'$, $R_3$ is $R_3'$, $R_{10}$ is $R_{10}'$, $R_{11}$ is $R_{11}'$, and X is X',

(xi) of (x) wherein Ro is Ro", $R_{10}$ is hydrogen, $R_{11}$ is $R_{11}''$, and X is (E)—CH=CH—,

(xii) of (xi) wherein Ro is Ro''', $R_2$ is $R_2''$, $R_3$ is hydrogen, $R_{11}$ is $R_{11}'''$, particularly a cation, and m is m',

(xiii) of (xii) wherein Ro is Ro'''' wherein $R_4'''$ is $R_4''''$, $R_2$ is hydrogen and m is m",

(xiv) of (xiii) wherein $R_{11}$ is a cation in particular sodium, potassium or ammonium, especially sodium,

(xv) of Group IBb wherein Ro is Ro', R and $R_1$ taken together are $—(CH_2)_m—$, $R_2$ is $R_2'$, $R_3$ is $R_3'$, $R_{10}$ is $R_{10}'$, and X is X',

(xvi) of (xv) wherein Ro is Ro", $R_{10}$ is hydrogen, and X is (E)—CH=CH—,

(xvii) of (xvi) wherein Ro is Ro''', $R_2$ is $R_2''$, $R_3$ is hydrogen, and m is m',

(xviii) of (xvii) wherein Ro is Ro'''' wherein $R_4'''$ is $R_4''''$, $R_2$ is hydrogen and m is m",

(xix)—(xxviii) of (i)—(v) and (x)—(xiv) wherein the hydroxy groups in the 3- and 5-positions of the group of formula IIa have the *erythro* configuration,

(xxix)—(xxxvi) of (vi)—(ix) and (xv)—(xviii) wherein $R_{10}$ and the hydrogen atom in the 6-position of the group of formula IIb are *trans* to each other, i.e. the *trans* lactones,

(xxxvii) of Group IAc wherein Ro is Ro', R is R', $R_1$ is $R_1'$, $R_2$ is $R_2'$, $R_3$ is $R_3'$ each $R_7$ is $C_{1-3}$alkyl or the two $R_7$'s taken together are $(CH_2)_2$ or $(CH_2)_3$, $R_{10}$ is $R_{10}'$, $R_{11}$ is $R_{11}'$ and X is X' with the proviso that X may be $—CH_2CH_2—$ only when $R_{10}$ is methyl,

(xxxviii) of (xxxvii) wherein Ro is Ro" each $R_7$ is $C_{1-2}$alkyl or the two $R_7$'s taken together are $(CH_2)_2$ or $(CH_2)_3$, $R_{10}$ is hydrogen, $R_{11}$ is $R_{11}''$ and X is (E)—CH=CH—,

(xxxix) of (xxxviii) wherein Ro is Ro", R is R", $R_1$ is $R_1''$, $R_2$ is $R_2''$, $R_3$ is hydrogen, each $R_7$ is $C_{1-2}$alkyl and $R_{11}$ is hydrogen or $C_{1-2}$alkyl, most preferably a cation,

(xl) of (xxxix) wherein Ro is Ro'''' wherein $R_4'''$ is $R_4''''$,R is $C_{1-2}$alkyl, $R_1$ is $C_{1-2}$alkyl, and $R_2$ is hydrogen,

(xli) of (xl) wherein $R_{11}$ is a cation, especially sodium, potassium or ammonium, particularly sodium,

(xlii) of Group IBc wherein Ro is Ro', R and $R_1$ taken together are $—(CH_2)_m—$, $R_2$ is $R_2'$, $R_3$ is $R_3'$, each $R_7$ is $C_{1-3}$alkyl or the two $R_7$'s taken together are $—(CH_2)_2$ or $_3—$, $R_{10}$ is $R_{10}'$, $R_{11}$ is $R_{11}'$, and X is X', with the provisos that $R_{11}$ may be hydrogen only when Q is —CO—, and X may be $—CH_2CH_2—$ only when $R_{10}$ is methyl,

(xliii) of (xlii) wherein Ro is Ro", each $R_7$ is $C_{1-2}$alkyl or both $R_7$'s taken together are $—(CH_2)_2$ or $_3—$, $R_{10}$ is hydrogen, $R_{11}$ is $R_{11}''$, and X is (E)—CH=CH—,

(xliv) of (xliii) wherein Ro is Ro''', $R_2$ is $R_2''$, $R_3$ is hydrogen, each $R_7$ is $C_{1-2}$alkyl, $R_{11}$ is $R_{11}'''$ (especially a cation), and m is m',

(xlv) of (xliv) wherein Ro is Ro'''' wherein $R_4'''$ is $R_4''''$, $R_2$ is hydrogen, and m is m",

(xlvi) of (xlv) wherein $R_{11}$ is a cation, particularly sodium, potassium or ammonium especially sodium and

(xlvii)—(lvi) of (xxxvii)—(xlvi) wherein Q is —CO—.

Groups (i)—(xviii) and (xxxvii)—(lvi) embrace each of the possible stereoisomers, racemates and mixtures of diastereoisomers. Groups (xix)—(xxviii) embrace the 3R,5S and 3S,5R isomers and the 3R,5S—3S,5R racemate of the compounds wherein X is (E)—CH=CH— having just two centres of asymmetry and the corresponding compounds having more than two centres of asymmetry, and Groups (xix) and (xxiv) also embrace the 3R,5R and 3S,5S isomers and the 3R,5R—3S,5S racemate of the compounds wherein X is $—CH_2CH_2—$ having just two centres of asymmetry and the corresponding compounds having more than two centres of asymmetry. Groups (xxix)—(xxxvi) embrace the 4R,6S and 4S,6R isomers and the 4R,6S—4S,6R racemate of the compounds wherein X is (E)—CH=CH— having just two centres of asymmetry and the corresponding compounds having more than two centres of asymmetry and Groups (xxix) and (xxxiii) also embrace the 4R,6R and 4S,6S isomers and the 4R,6R—4S,6S racemate of

the compounds wherein X is $-CH_2CH_2-$ having just two centres of asymmetry and the corresponding compounds having more than two centres of asymmetry.

A particular compound group covers those compounds of formula I wherein Ro represents ring A

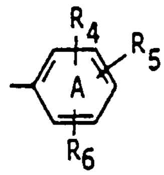

R is hydrogen or primary or secondary $C_{1-6}$alkyl not containing an asymmetric carbon atom, and

$R_1$ is primary or secondary $C_{1-6}$alkyl not containing an asymmetric carbon atom or

R and $R_1$ taken together are $-(CH_2)_m-$ or (Z)$-CH_2-CH=CH-CH_2-$, wherein m is 2, 3, 4, 5 or 6,

$R_2$ is hydrogen, $C_{1-3}$alkyl, *n*-butyl, *i*-butyl, *t*-butyl, $C_{1-3}$alkoxy, *n*-butoxy, *i*-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the proviso that not more than one of $R_2$ and $R_3$ is trifluoromethyl, not more than one of $R_2$ and $R_3$ is phenoxy, and not more than one of $R_2$ and $R_3$ is benzyloxy,

$R_4$ is hydrogen, $C_{1-3}$alkyl, *n*-butyl, *i*-butyl, *t*-butyl, $C_{1-3}$alkoxy, *n*-butoxy, *i*-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_6$ is hydrogen, $C_{1-2}$alkyl, $C_{1-2}$alkoxy, fluoro or chloro, with the provisos that not more than one of $R_4$ and $R_5$ is trifluoromethyl, not more than one of $R_4$ and $R_5$ is phenoxy, and not more than one of $R_4$ and $R_5$ is benzyloxy,

X is $-(CH_2)_n-$ or (E)$-CH=CH-$, wherein n is 1, 2 or 3, and

(a) Z 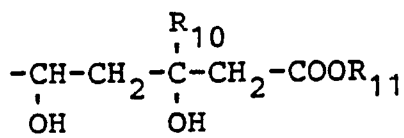 or 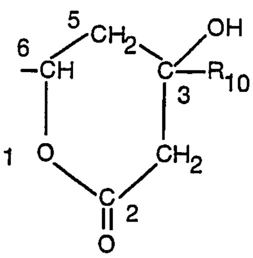 (b)

wherein

$R_{10}$ is hydrogen or $C_{1-3}$alkyl, and

$R_{11}$ is hydrogen, $R_{12}$ or M,

wherein

$R_{12}$ is a physiologically acceptable and hydrolyzable ester group, and

M is a pharmaceutically acceptable cation.

The compounds of formula I may be prepared by the following reactions wherein Ind stands for

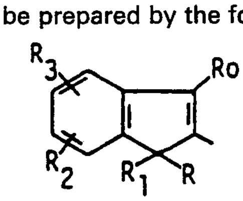

and substituents are as defined above.

a) when X is $(CH_2)_n$ or (E)$-CH=CH-$ and $R_{10}$ is hydrogen reducing a compound of formula IV

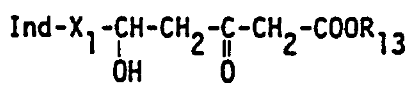 IV

wherein $R_{13}$ is a radical forming an ester, and $X_1$ is $(CH_2)_n$ or (E)$-CH=CH-$,

b) when X is $(CH_2)_n$ or (E)$-CH=CH-$ and $R_{10}$ is $C_{1-3}$alkyl hydrolysing a compound of formula XII

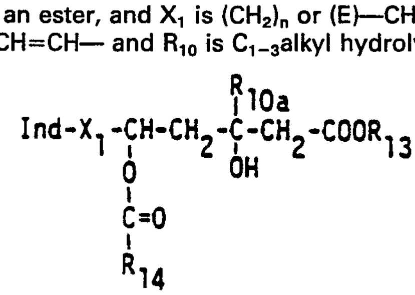 XII

wherein $R_{10a}$ is $C_{1-3}$alkyl, $R_{14}$ is part of an ester forming group and $X_1$ and $R_{13}$ are as defined above,

c) when X is —CH=CH— or —$CH_2$—CH=CH— and IIb is in 4R,6S configuration or X is —$CH_2CH_2$ or $CH_2CH_2CH_2$ and IIb is in 4R,6R configuration deprotecting a compound of formula XXXIX

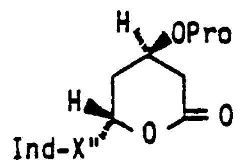

XXXIX

wherein X″ represents —$CH_2CH_2$, —$CH_2CH_2CH_2$, —CH=CH— or —$CH_2$CH=CH— and Pro is a protecting group,

d) when X is —$(CH_2)_2$—, —$(CH_2)_3$—, —$(CH_2)_q$CH=CH$(CH_2)_q$— deprotecting a compound of formula XXXII

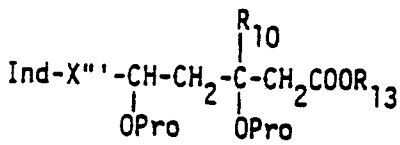

XXXII

wherein X‴ is —$(CH_2)_2$—, —$(CH_2)_3$— or —$(CH_2)_q$—CH=CH—$(CH_2)_q$—, and q, $R_{10}$, $R_{13}$ and Pro are as defined above,

e) when Q is

$$\underset{\overset{\|}{O}}{-C-}$$

oxidising the corresponding compound of formula I wherein Q is

$$\underset{\overset{|}{OH}}{-CH-}$$

f) when

Q is 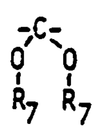

and II is in ester form ketalising the corresponding compound of formula I wherein Q is

$$\underset{\overset{\|}{O}}{-C-}$$

g) hydrolysing a compound of formula I in the form of an ester or a lactone or

h) esterifying or lactonising a compound of formula I in free acid form, and when a free carboxyl group is present, recovering the compound obtained in free acid form or in the form of a salt.

$R_{13}$ is preferably $C_{1-3}$alkyl, n-butyl, i-butyl, t-butyl or benzyl, more preferably $C_{1-3}$alkyl and especially $C_{1-2}$alkyl and $R_{14}$ is preferably $C_{1-3}$alkyl more preferably n-$C_{1-3}$alkyl, especially $C_{1-2}$alkyl.

Process a) is particularly suited for compounds wherein X is —$(CH_2)_n$— or (E)—CH=CH and in ester form.

Process b) is particularly suited for compounds wherein X is —$(CH_2)_n$— or (E)—CH=CH in salt form.

Process c) is particularly suited for compounds wherein X is —(E)—CH=CH— and the lactone is in 4R,6S configuration and those wherein X is —$CH_2CH_2$— and the lactone is in 4R,6R configuration.

Process d) is particularly suited for compounds in ester form.

It will readily be appreciated that the various forms of the compounds of formula I may be interconverted as indicated in g) and h) above, whereby lactonisation may only take place when Q is

$$\underset{\overset{|}{OH}}{-CH-}$$

and ketals cannot be isolated in free acid form or esterified.

In the same way compounds obtained according to a) to f) may be as appropriate hydrolysed to free acid forms and free acid forms may be esterified or lactonised to produce a desired end-product. The invention thus also provides a process for preparing a compound of formula I which comprises hydrolysing a compound of formula I in ester or lactone form or esterifying or lactonising a compound of formula I in free acid form and when a free carboxyl group is present recovering the compound obtained in free acid form or in the form of a salt.

Unless otherwise stated reactions are performed in a manner conventional for the type of reaction involved. Molar ratios and reaction times are as a rule conventional and non-critical and are chosen according to principles well established in the art on the basis of reactions and conditions employed.

Solvents, alone or as mixtures, are generally chosen which remain inert and liquid during the reaction in question.

Examples of inert atmospheres are usually nitrogen or a nobel gas, nitrogen being preferred. Most reactions, including those wherein use of an inert atmosphere is not mentioned, are carried out under such for convenience.

EP 114027 and 117228 including the examples thereof disclose analogous processes and further suitable reaction conditions.

Reduction according to a) is preferably carried out using a mild reducing agent such as sodium borohydride or, a complex of t-butylamine and borane in an inert organic solvent such as a lower alkanol, preferably ethanol, conveniently at a temperaturee of −10°C to 30°C, under an inert atmosphere.

Use of an optically pure starting material will lead to only two optical isomers (diastereoisomers) of the resulting end product. However, if stereospecificity is desired it is preferred to utilize a stereo-selective reduction in order to maximize production of a mixture of the *erythro* stereoisomers (racemate) of which the preferred stereoisomer (as set forth above) is a constituent. Stereoselective reduction is preferably carried out in three steps. For example in the first step, the ketoester of formula IV is treated with a tri(primary or secondary $C_{2-4}$alkyl)borane, preferably triethylborane or tri-n-butylborane, and optionally air to form a complex. The reaction temperature is suitably 0° to 50°C, preferably 0° to 25°C. The first step is carried out in an anhydrous inert organic solvent, preferably an ether solvent such as tetrahydrofuran diethyl ether, 1,2-dimethoxyethane or 1,2-diethoxyethane, with tetrahydrofuran, being the most preferred solvent especially in a 3—4:1 mixture with methanol when pure *erythro* product is desired. In the second step, the complex is reduced with sodium borohydride, preferably in the same solvent as utilized for the first step, at −100° to −40°C, preferably −100° to −70°C. In the third step, the product of the second step is, for example, treated with, preferably, anhydrous methanol at 20° to 40°C, preferably 20° to 25°C. The amount of methanol is not critical. However, a large excess, e.g. 50—500 moles per mole of ketoester of formula IV is typically utilized. Alternatively a mixture of methanol, e.g. 30% aqueous $H_2O_2$ and a pH 7—7.2 aq. phosphate buffer is used.

Hydrolysis according to b) or g) is carried out in a manner conventional for such reactions e.g. employing an inorganic hydroxide such as NaOH or KOH with, if desired subsequent acidification to give the free acid form. Suitable solvents are mixtures of water and water miscible solvents such as lower alkanols e.g. methanol or ethanol and reaction conveniently takes place at temperatures from 0°C to reflux preferably 0° to 75°C e.g. 20° to 70°C. If it is desired to recover the compound in a salt form corresponding to the cation of the hydroxide employed then slightly less than equivalent amounts of the latter may be employed. In b) $R_{14}$ will conveniently be the same as $R_{13}$ e.g. $C_{1-3}$alkyl more preferably n-$C_{1-3}$alkyl, especially $C_{1-2}$.

Oxidation according to e) can be carried out when X is —CH=CH— or —$CH_2$CH=CH— using activated $MnO_2$ at 20° to 80°C, preferably 40° to 80°C in an anhydrous inert organic solvent such as an ether solvent e.g. $(C_2H_5)_2O$, 1,2-diethoxyethane, 1,2-dimethoxyethane, tetrahydrofuran and mixtures thereof, or when X is $(CH_2)_n$ or —CH=CH—$CH_2$ using Swerns reagent (oxalyl chloride + dimethylsulfoxide) with triethylamine in e.g. —$CH_2Cl_2$ at −60° to 40°C preferably −50°C.

Ketalisation according to f) can be carried out in the case of open chain ketals using H—C$(OR_7)_3$ in the presence of catalytic amounts of pyridinium p-toluene sulfonate and a hydrocarbon solvent, for example benzene, toluene, xylene and mixtures thereof, halogenated lower alkane solvent, for example carbon tetrachloride, chloroform, 1,1-dichloroethane, 1,2-dichloroethane, methylene chloride and 1,1,2-trichloroethane, usually preferably methylene chloride or benzene at 20—25°C or for cyclic ketals using HO—$(CH_2)_{2-3}$—OH under the same conditions.

Lactonisation according to h) is carried out in conventional manner e.g. by heating the corresponding acid in anhydrous inert organic solvent e.g. a hydrocarbon such as benzene, toluene or a xylene or mixtures thereof, preferably at temperatures of 75°C to reflux although more preferably not above 150°C. Preferably, however, a lactonisation agent, e.g. a carbodiimide, preferably a water-soluble carbodiimide such as N-cyclohexyl-N′-[2′-(methylmorpholinium)ethyl]carbodiimide p-toluenesulfonate, in an anhydrous inert organic solvent, e.g. a halogenated lower alkane, preferably methylene chloride is employed. Reaction temperatures then lie typically between 10° and 35°C, especially 20° to 25°.

As is evident to those in the art, a racemic *threo* 3,5-di-hydroxycarboxylic acid yields a racemic *cis* lactone (two stereoisomers) and a racemic *erythro* 3,5-dihydroxycarboxylic acid yields a racemic *trans* lactone (two stereoisomers). Likewise if a single enantiomer of the 3,5-dihydroxycarboxylic acid is utilized,

a single enantiomer of the lactone is obtained. For example, lactonisation of a 3R,5S *erythro* dihydroxycarboxylic acid yields a 4R,6S lactone.

Esterification according to h) is conventional, employing e.g. a large excess of a compound $R_{13}OH$, wherein $R_{13}$ is as defined above at e.g. 20°C to 40°C in the presence of a catalytic amount of an acid such as p-toluenesulfonic acid. Direct esterification is particularly suited when Q is

$$-\underset{\underset{O}{\|}}{C}-.$$

Preferably, however, esterification takes place by first forming the corresponding lactone and reacting this with $M_2^{\oplus}\ {}^{\ominus}OR_{13}$ ($M_2^{\oplus}=Na^{\oplus}$ or $K^{\oplus}$) at 0° to 70°C preferably 20° to 25°C in an inert organic solvent e.g. an ether such as tetrahydrofuran or an alcohol of formula $R_{13}OH$ if a liquid.

Examples of protecting groups in reaction c) and d) are diphenyl-t-butylsilyl, tri-isopropylsilyl or dimethyl-t-butylsilyl, benzyl, triphenylmethyl, tetrahydrofuran-2-yl, tetrahydropyran-2-yl, 4-methoxytetra-hydrofuran-4-yl, $C_{2-6}$n-alkanoyl. Especially preferred are trisubstituted silyl radicals in particular diphenyl-t-butylsilyl (=Pro').

Deprotection is carried out in conventional manner e.g. by cleavage under mild conditions such as employing e.g. for removal of a diphenyl-t-butylsilyl a fluoride reagent e.g. tetra-n-butylammonium fluoride in an anhydrous inert organic medium preferably tetrahydrofuran containing glacial acetic acid at temperatures of 20° to 60°C, especially 20° to 25°C. Preferably 1—4 moles of fluoride per mole protecting group are used with 1—2 moles, preferably 1.2 to 1.5 mmoles of glacial acetic acid to each mole of fluoride.

The required starting materials may be prepared for example as illustrated in the following reaction schemes or in the examples hereinafter.

Further suitable reaction conditions are disclosed e.g. in EP 114027 and 117228 including the examples thereof.

Abreviations:

| | |
|---|---|
| AIO | — anhydrous inert organic solvent |
| ES | — ether solvent e.g. diethylether, 1,2-diethoxyethane, 1,2-dimethoxyethane, THF or mixtures thereof |
| HC | — hydrocarbon solvent e.g. benzene, toluene, xylene or mixtures thereof |
| HLA | — halogenated lower alkane solvent e.g. $CCl_4$, $CHCl_3$, 1,1-dichloroethane, 1,2-dichloroethane, methylene chloride, 1,1,2-trichloroethane, preferably $CH_2Cl_2$ |
| IO | — inert organic solvent |
| THF | — tetrahydrofuran |
| LDA | — lithiumdiisopropylamide |
| nBuLi | — n-butyllithium |
| DMF | — dimethylformamide |
| DIBAH | — diisobutylaluminium hydride |

Variables not previously defined

$R_{15}$ is $C_{1-2}$alkyl, preferably methyl

$X_2$ is $CH_2$ or $(CH_2)_2$

$X_3$ is a direct bond or $CH_2$

$X_4$ is —CH=CH—, —CH=CH—$CH_2$— or —$CH_2$—CH=CH— preferably (E)—CH=CH—, (E)—CH=CH—$CH_2$— or (E)—$CH_2$—CH=CH— especially (E)—CH=CH—,

$X_5$ is $(CH_2)_2$— or $(CH_2)_3$— especially —$(CH_2)_2$—

$X_6$ is —CH=CH— or —$CH_2$—CH=CH—, preferably CH—CH and especially (E)—CH=CH—

Y is Cl, Br or I

Y' is Cl or Br

Lac$_1$ = 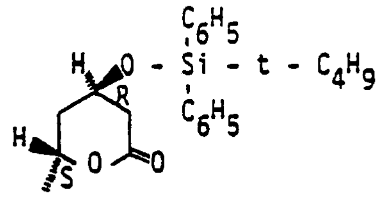 Lac$_2$ = as Lac$_1$ but in 4R,6R configuration

Pro' = 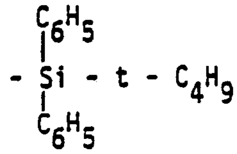

$R_{13}'$ is $C_{1-3}$alkyl, n-butyl, i-butyl, t-butyl or benzyl each $R_{16}$ is independently $CH_3$ or $C_2H_5$ and they are preferably the same

$R_{17}$ is $R_1$, $(CH_2)_m$—Y' or (Z)—$CH_2$—CH=CH—$CH_2$—Y'

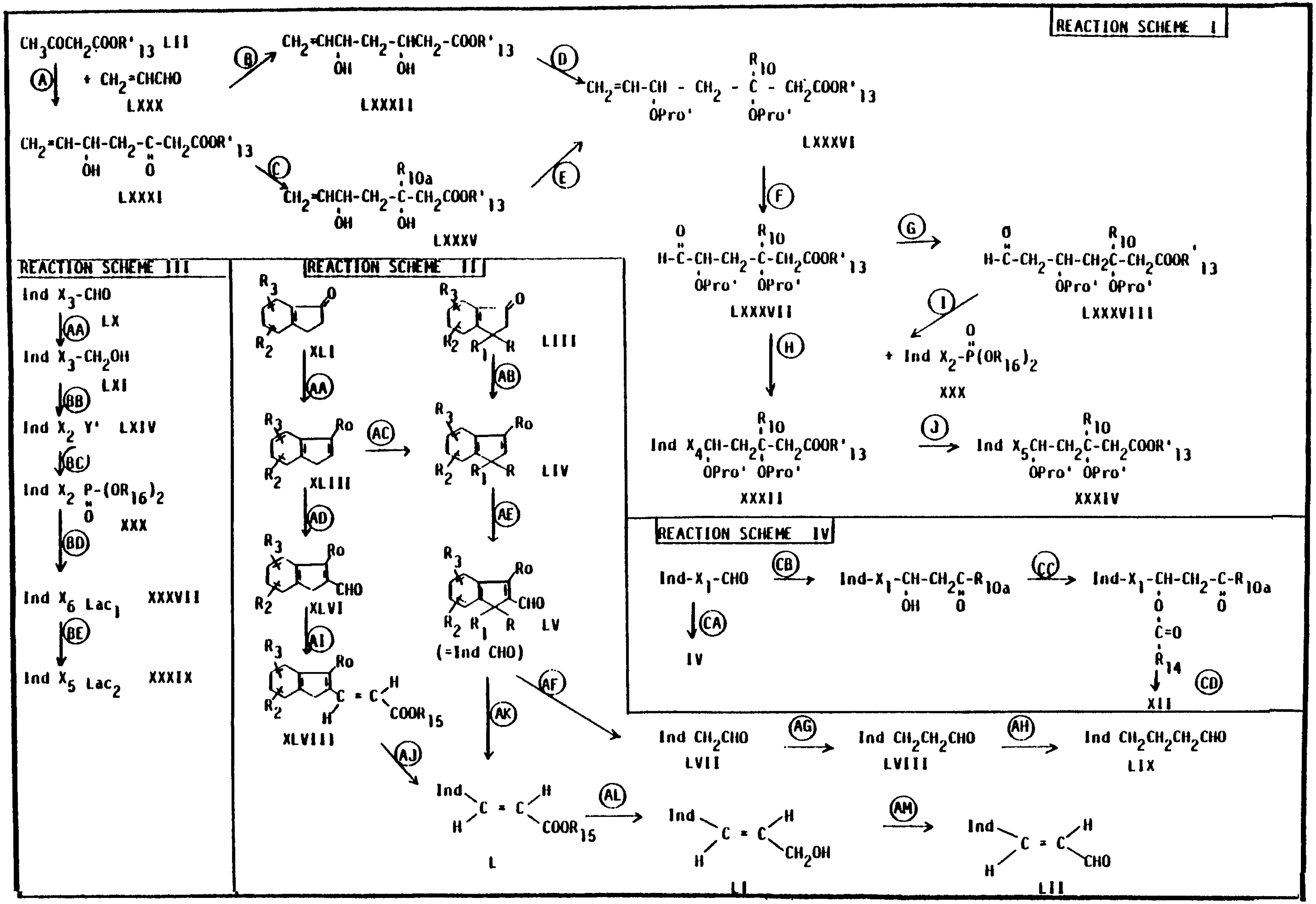
REACTION SCHEME I
A
B
C
D
E
F
G
H
I
J
LII
LXXX
LXXXI
LXXXII
LXXXV
LXXXVI
LXXXVII
LXXXVIII
XXX
XXXII
XXXIV
REACTION SCHEME III
AA
BB
BC
BD
BE
LX
LXI
LXIV
XXX
XXXVII
XXXIX
REACTION SCHEME II
AA
AB
AC
AD
AE
AF
AI
AJ
AK
AL
AG
AH
AM
XLI
LIII
XLIII
LIV
XLVI
LV
(=Ind CHO)
XLVIII
L
LI
LII
LVII
LVIII
LIX
REACTION SCHEME IV
CA
CB
CC
CD
IV
XII

| REACTION | TYPE / STEPS | SPECIAL CONDITIONS/REACTIONS | TEMPERATURE | ATMOS. | SOLVENT |
|---|---|---|---|---|---|
| (A) | | 1. Strong base e.g. LDA or NaH followed by n-BuLi to generate dianion<br>2. Add LII<br>3. Quench with e.g. $NH_4Cl$ | ① -50° to 10° pref. -10° to 10°<br>② -80° to 0°, pref. -40° to -20° esp. -35° to -30° rising to 20°-25°<br>③ -80° to 25° | Inert | AIO e.g. ES pref. THF |
| (B) | Reduction | As process a) above | → | → | → |
| (C) | Grignard | $R_{10a}MgY$ (LXXXIV)<br>quench on completion e.g. with $NH_4Cl$ | -70° to 25° pref.<br>-50° to 0° | Inert | as A |
| (D)(E) | Silylation | 2-8 moles pref. 4 moles of Pro'Cl per mole<br>LXXXII or LXXXV + 2 moles<br>of imidazole per mole Pro'Cl | 20° to 30° pref.<br>20 to 25° | Inert | anh. DMF |
| (F) | Ozonolysis | $O_3$ in excess; then quench with<br>$(CH_3)_2S$ or $(C_6H_5)_3P$ | -80° to -70°<br>pref. -78° | - | $C_{1-3}$alkanol esp. $CH_3OH$<br>or HLA esp. $CH_2Cl_2$ or<br>$CH_3COOC_2H_5$ |
| (G) | Wittig | ① $(C_6H_5)_3\overset{\oplus}{P}-CH_2OCH_3Cl^{\ominus}$ (LVI)<br>+ strong base e.g. NaH, phenyl lithium or nBu-Li | 1. -40 to 0° pref.<br>-35° to -20° | Inert | as A |
| | | ② Add LXXXVII | 2. -30° to 0° pref.<br>-20° to 0° | " | " |
| | | ③ hydrolysis: excess of strong acid e.g. aq. perchloric | 3. 0° to 30° | - | e.g. acid + ES e.g. aq. perchloric + THF |

| REACTION | TYPE / STEPS | SPECIAL CONDITIONS/REACTIONS | TEMPERATURE | ATMOS. | SOLVENT |
| --- | --- | --- | --- | --- | --- |
| (H), (I) | | ① strong base e.g. nBuLi or LDA | 1) -10° to 0° | Inert | as (A) |
| | | ② add LXXXVII or LXXXVIII | 2) -10° to 0° | " | as (A) |
| (J) | Hydrogenation | $H_2$ at raised pressure (e.g. 30-60 psi) $PtO_2$ as catalyst | 20° to 25° | - | Loweralkanol e.g. $C_2H_5OH$ |
| (AA), (AB) | Grignard + dehydration | ① Ro-MgY (XLII) + opt. trace of $CH_3I$ or 1,2-dibromo-ethane | 10° to reflux pref. 30° to 38° in $(C_2H_5)_2O$ and 35° to 65° in THF | Inert | AIO e.g. ES esp. THF or $(C_2H_5)_2O$ |
| | | ② add XLI or LIII | 20° to 25° | " | " " |
| | | ③ e.g. with glacial $CH_3COOH$ | 90° to, esp. 100° to, pref. 100° to reflux | - | Neat |
| | | or HCl | 0° to 25° esp.10-25° | | |
| (AC), (AJ) | Alkylation | ① generation of mono- or di-carbanion with NaH | -5° to 5° pref. 0° | Inert | AIO pref. ES e.g. THF or HC e.g. toluene pref. toluene |
| | | ② when R is H and $R_1$ is alkyl or together they are $(CH_2)_m$- or (Z)-$CH_2$-CH=CH-$CH_2$ 1-1.05 moles of $R_{17}Y'$; when R and $R_1$ identical alkyl 2-2.1 moles. For different alkyls as R, $R_1$ repeat reaction | 0° to 25° | " | " " |

| REACTION | TYPE / STEPS | SPECIAL CONDITIONS/REACTIONS | TEMPERATURE | ATMOS. | SOLVENT |
|---|---|---|---|---|---|
| (AD), (AE) | Vilsmeier-Haack | (1) $C_6H_5-\underset{CH_3}{N}CHO + POCl_3$ | 0° to 35°, pref. 20° 25° | Inert | Acetonitrile or neat |
| | | (2) add XLIII or LIV | 10° to 30° pref. 10° rising to 20° to 25° | " | " " |
| | | (3) hydrolysis ($H_2O$) | 0° to 25° | - | water |
| (AF), (AG) (AH) | Wittig | as (G) | → | → | → |
| (AI), (AK) | Wittig | $(C_6H_5)_3P = CH-COOR_{15}$ | 50° to reflux pref. 60° to 115° esp. 90° to 115° | " | as (AC) |
| (AL) | Reduction | Strong metal hydride e.g. $LiAlH_4$ or DIBAH | -80° to 25° pref. -80° to 0° esp. -80° to -70° | " | AIO pref. ES e.g. THF; HLA esp. $CH_2Cl_2$ or HLA + toluene |
| (AM) | Oxidation | excess activated $MnO_2$ | 20° to 30° pref. 20° to 25° | | IO pref. HLA esp. $CH_2Cl_2$ or HC esp. toluene |
| (BA) | Reduction | non-stereospecific as is a) | → | → | → |
| (BB) | Halogenation | $SOY'_2$ or $PY'_3$ | -10° to 80° | - | AIO pref. ES eq. $(C_2H_5)_2O$ or THF; HLA e.g. $CH_2Cl_2$; or HC e.g. benzene |
| (BC) | | add $P(OR_{16})_3$ | 20° to 140° usually 110° to 140° | yes | HC e.g. benzene or xylene or neat with excess $P(OR_{16})_3$ |
| (BD) | Wittig | as (H) | → | → | → |
| (BE) | Hydrogenation | as (J) | → | → | → |

The conditions given hereinabove are largely conventional for such reactions and can be varied in conventional manner according to the particular intermediates/end products. This applies e.g. to molar ratios, temperature, reaction times and the like which are chosen according to principles well established in the art on the basis of reactants and conditions employed.

Intermediates, the production of which is not described above, are either known or may be prepared according to or analogously to known methods e.g. as described in EP 114027 and 117228 including the examples thereof. Process CA to CD are for example described in EP 114027.

Reaction products, both intermediates and final, can be isolated (e.g. from compound mixtures or reaction mixtures) and purified in conventional manner whereby intermediates can, where appropriate, be employed directly in a subsequent reaction.

Mixtures of stereoisomers (*cis, trans* and optical) can be separated by conventional means at whatever stage of synthesis is appropriate. Such methods include re-crystallisation, chromatography, formation of esters with optical pure acids and alcohols or of amides and salts with subsequent reconversion under retention of optical purity. For example diastereoisomeric (−)-α-naphthylphenylmethylsilyl derivatives of a lactone type end product of formula I may be separated by conventional means.

Salts may be prepared in conventional manner from free acids, lactones and esters and vice-versa. In some cases e.g. for groups IAc, IBc in ketal form ion-exchange may be required for salt formation. Whilst all salts are covered by the invention pharmaceutically acceptable salts especially sodium, potassium and ammonium particularly sodium salts are preferred.

The various forms of the compounds of formula I are by virtue of their interconvertability useful as intermediates in addition to the use set out below.

Also within the scope of this invention are the intermediates of formulae IV, XII, XXXII, XXXIV, XXXVII, XXXIX, XLVI, XLVIII, L-LII, LV, LVII—LIX, LX, LXI, LXIV and products of reactions CB and CC.

The preferences for each variable are the same as set out for formula I and preferred groups of compounds correspond to those listed for formula I as appropriate to and consistent therewith.

The compounds of formula I possess pharmacological activity in particular as competitive inhibitors of 3-hydroxy-3-methyl-glutaryl coenzyme A (HMG—CoA) reductase and as a consequence are inhibitors of cholesterol biosynthesis as demonstrated in the following tests.

Test A: In Vitro Microsomal Assay of HMG—CoA Reductase Inhibition: (dosage range 0.0001—2000 μmol):

200 μl aliquots (1.08—1.50 mg./ml.) of rat liver microsomal suspensions, freshly prepared from male Spargue-Dawley rats (150—225 g. body weight), in Buffer A with 10 mmol. dithiothreitol are incubated with 10 μl. test substance dissolved in dimethylacetamide and assayed for HMG—CoA reductase activity as described by Ackerman et al., J. Lipid Res. *18,* 408—413 (1977). In the assay the microsomes are the source of the HMG—CoA reductase enzyme which catalyses the reduction of HMG—CoA to mevalonate. The assay employs a chloroform extraction to separate the product, [$^{14}$C]mevalonolactone, formed by the HMG—CoA reductase reaction from the substrate, [$^{14}$C]MHG—CoA. [$^{3}$H]mevalono-lactone is added as an internal reference. Inhibition of MHG—CoA reductase is calculated from the decrease in specific activity [$^{14}$C/$^{3}$H]mevalonate) of test groups compared to controls.

Test B: In Vivo Cholesterol Biosynthesis Inhibition: (dosage range 0.01—200 mg/kg):

In vivo studies utilize male Wistar Royal Hart rats weighing 150±20 g which have been kept for 7—10 days on an altered light cycle (6:30 a.m. - 6:30 p.m. dark) housed two per cage and fed powdered Purina Rat Chow and water *ad libitum.* Three hours before the diurnal maximum of cholesterol synthesis at mid-dark, the rats are administered the test substances dissolved or as a suspension in 0.5% carboxymethylcellulose in a volume of 1 ml/100 g body weight. Controls receive vehicle alone. One hour after receiving the test substance, the rats are injected intraperitoneally with about 25 μCi/100 g body weight of sodium [1-$^{14}$C] acetate 1—3 mCi/mmol. Two hours after mid-dark, blood samples are obtained under sodium hexobarbitol anesthesia and the serum separated by centrifugation.

Serum samples are saponified and neutralized, and the 3β-hydroxy sterols are precipitated with digitonin basically as described by Sperry et al., J. Biol. Chem. *187,* 97 (1950). The [$^{14}$C]digitonides are then counted by liquid scintillation spectrometry. After correcting for efficiencies, the results are calculated in nCi (nanocuries) of sterol formed per 100 ml of serum. Inhibition of sterol synthesis is calculated from the reduction in the nCi of sterols formed from test groups compared to controls.

The compounds are thus indicated for use as hypolipoproteinemic and anti-atherosclerotic agents.

An indicated suitable daily dosage for use in the treatment of hyperlipoproteinemia and atherosclerosis is from about 1 to 2000 mg preferably 1 to 150 mg suitably administered one to four times daily or in controlled release form. A typical dosage unit for oral administration may contain 0.25 to 500 mg.

The compounds of formula I may be administered in similar manner as known compounds suggested for use in such indications e.g. Compactin or Mevinolin. The suitable daily dosage for a particular compound will depend a number of factors such as its relative potency of activity. It has, for example been determined that the preferred compound (compound of example no. 2) obtained an $ED_{50}$ of 0.07 mg/kg in Test B compared with 3.5 mg/kg for Compactin and 0.41 mg/kg for Mevinolin. It is therefore indicated that the compounds may be administered at similar or significantly lower dosages (e.g. 1—30 mg/d) than conventionally proposed e.g. for Compactin.

The invention therefore also concerns a method of treating hyperlipoproteinemia or atherosclerosis by administration of a compound of formul I in free acid form or in the form of a physiologically-acceptable ester or a lactone thereof or in pharmaceutically acceptable salt form as well as such compounds for use as pharmaceuticals e.g. as hypolipoproteinemic and anti-atherosclerotic agents.

The compounds may be administered alone, or in admixture with a pharmaceutically acceptable diluent or carrier, and, optionally other excipients, and administered orally in such forms as tablets, elixirs, capsules or suspensions or parenterally in such forms as injectable solutions or suspensions.

The preferred pharmaceutical compositions from the standpoint of ease of preparation and administration are solid compositions, particularly tablets and hard-filled or liquid-filled capsules.

Such compositions also form part of the invention.

The following references illustrate the background art: Sandoz EP 114027, Sandoz EP 117228, Merck EP 113881, Sankyo EP 11928 and G. E. Stokker et al., *J. Med. Chem. 28* (March 1985) 347—358; they all pertain to HMG—CoA reductase inhibiting compounds structurally similar to the compounds of the present invention.

The compounds of the present invention possess advantageous effects over, e.g., the compounds of EP 117228.

The following Examples, in which all temperatures are in °C illustrate the invention.

Example 1

Ethyl *erythro*-(E)-3,5-dihydroxy-7-[3'-(4''-fluorophenyl)-spiro[cyclopentane-1,1'-(1H)-inden]-2'-yl]hept-6-enoate (Cmpd. No. 1)

*Step 1* (Reaction AA)

*3-(4'-Fluorophenyl)-1H-indene (Compound XLIIIa)*

A solution of 5.1 g (39 moles) of 1-indanone in 15 ml of anhydrous diethyl ether is added over a 30 minute period to a solution of 4-fluorophenylmagnesium bromide (prepared from 7.97 g (45.5 mmoles) of 1-bromo-4-fluorobenzene, 1.33 g (54.7 mmoles) of magnesium turnings and a trace of iodine in 25 ml of anhydrous diethyl ether) stirred at 20°—25°C under nitrogen. The reaction mixture is stirred at 20°—25°C under nitrogen for 16 hours and quenched with saturated ammonium chloride solution. The organic phase is separated, dried over anhydrous sodium sulfate and evaporated at reduced pressure, and the residual oil is dissolved in 16 ml of glacial acetic acid.

The obtained solution is refluxed for 15 minutes, and the acetic acid is evaporated at reduced pressure. The residue is flash chromatographed on a silica gel column utilizing 10% ethyl acetate/petroleum ether as the eluant, and the eluant is evaporated at reduced pressure to obtain a solid which is recrystallized from 95% ethanol to obtain the product, m.p. 38—40°C.

*Step 2* (Reaction Ad)

*3-(4'-Fluorophenyl)-1H-indene-2-carboxaldehyde (Cmpd. XLVIa)*

5 ml of acetonitrile is added to a mixture of 0.973 ml (10 mmoles) of phosphorus oxychloride and 1.3 ml (10 mmoles) of N-methylformanilide stirred at 20°—25°C, the reaction mixture is stirred at 20°—25°C for 30 minutes and cooled to 5°C, a solution of 2 g (9.5 mmoles) of XLIIIA in 5 ml of acetonitrile is added dropwise with stirring, and the reaction mixture is stirred at 20°—25°C for 6.5 hours, the reaction mixture being maintained under nitrogen throughout. The reaction mixture is poured onto ice and extracted several times with 4:1 diethyl ether/petroleum ether. The extracts are combined, washed with water, washed with saturated sodium chloride solution, dried over anhydrous sodium sulfate, passed through a short silica gel column and evaporated at reduced pressure to obtain the crude product as an oil. The oil is dissolved in chloroform and flash chromatographed on a silica gel column, the eluant is evaporated at reduced pressure, and the residue is crystallized from petroleum ether to obtain the product, m.p. 70°—71°C.

*Step 3* (Reaction AI)

*Methyl (E)-3-[3'-(4''-fluorophenyl)-1H-inden-2'-yl]propenoate (Compound XLVIIIa)*

A solution of 573 mg (2.42 mmoles) of Compound XLVIa and 1.01 g (2.91 mmoles) of (carbomethoxymethylene)triphenylphosphorane in 6 ml of dry toluene is refluxed under nitrogen for 7 hours. The reaction mixture is cooled to 20°—25°C, diethyl ether is added, and the mixture is filtered through a short silica gel column. The eluate is evaporated at reduced pressure to obtain a yellow oil which is crystallized from 95% ethanol to obtain the product, m.p. 121°—122°C.

*Step 4* (Reaction AJ)

*Methyl (E)-3-[3'-(4''-fluorophenyl)spiro[cylopentane-1,1'(1H)-inden]-2'-yl]propenoate (Compound La)*

112 mg (2.3 mmoles) of sodium hydride (as a 50% by weight dispersion in mineral oil) is added to a solution of 340 mg (1.16 mmoles) of compound XLVIIIa in 5 ml of dry dimethylformamide stirred at 0°C, the reaction mixture is stirred at 0°C for 10 minutes, 0.143 ml (1.16 mmoles) of 1,4-dibromobutane is added dropwise with stirring over a 5 minute period, and the reaction mixture is allowed to gradually warm to 20°—25°C with stirring and stirred at 20°—25°C for 16 hours, the reaction mixture being maintained under nitrogen throughout. The reaction mixture is diluted with diethyl ether, dilute hydrochloric acid is added, and the mixture is extracted three times with diethyl ether. The diethyl ether extracts are combined,

washed with water, washed with saturated sodium chloride solution, dried over anhydrous sodium sulfate, filtered and evaporated to dryness at reduced pressure. The residue is chromatographed on a silica gel column utilizing 4:1 petroleum ether/acetone as the eluant to obtain the product, m.p. 143°—145°C.

*Step 5* (Reaction AL)
*(E)-3-(3'-(4''-fluorophenyl)spiro[cyclopentane-1,1'(1H)-inden]-2'-yl]-prop-2-en-1-ol (Compound LIa)*

2 ml of 1.5M diisobutylaluminium hydride/toluene (3 mmoles) is added dropwise to a solution of 220 mg (0.632 mmole) of Compound La in 4 ml of dry methylene chloride stirred at −78°C under nitrogen, and the reaction mixture is stirred under the same conditions for 20 minutes, quenched with dilute hydrochloric acid and extracted several times with methylene chloride. The methylene chloride extracts are combined, washed with water, washed with saturated sodium chloride solution, dried over anhydrous sodium sulfate, filtered and evaporated at reduced pressure to obtain the product which solidifies upon standing, m.p. 102°—104°C.

*Step 6* (Reaction AM)
*(E)-3-[3'-(4''-fluorophenyl)spiro[cyclopentane-1,1'(1H)-inden]-2'-yl]-prop-2-enal (Compound LIIa)*

300 mg (3.45 mmoles) of activated manganese dioxide is added to a solution of 170 (0.531 mmole) of Compound LIa in 4 ml of dry toluene stirred at 20°—25°C, and the reaction mixture is stirred at 20°—25°C under nitrogen for 24 hours, filtered to remove the manganese dioxide and evaporated at reduced pressure to obtain the yellow product which solidifies upon standing, m.p. 123—125°, following recrystallisation from diethylether/petroleum ether, 129°—130°C.

*Step 7* (Reaction CA)
*Ethyl (E)-7-[3'-(4''-fluorophenyl)spiro[cyclopentane-1,1'(1H)-inden]-2'-yl]-5-hydroxy-3-oxohept-6-enoate (Compound IVa)*

(a) A stock solution of the dianion of ethyl acetoacetate is prepared as follows: 7.5 ml of 1.6M *n*-butyllithium/hexane (12.0 mmoles) is added over a period of 5 minutes to a solution of 1.23 g (12.2 mmoles) of diisopropylamine in 25 ml of dry tetrahydrofuran stirred at −5°—0°C under nitrogen, the rate of addition being such that the temperature does not exceed 5°C. The reaction mixture is stirred at −30°C for 15 minutes under nitrogen, 780.8 mg (6 mmoles) of ethyl acetoacetate (dried over molecular sieves) is slowly added, and the reaction mixture is stirred at −30° to −20°C under nitrogen for 45 minutes.

(b) 3.3 ml of the stock solution of the dianion of ethyl acetoacetate of Part (a) (0.6 mmoles) is added to a solution of 126 mg (0.396 mmole) of Compound LIIa in 3 ml of dry tetrahydrofuran stirred at −60°C under nitrogen, and the reaction mixture is stirred under the same conditions for 1 hour, quenched with water, acidified with dilute hydrochloric acid and extracted three times with ethyl acetate. The ethly acetate extracts are combined, washed with water, washed with saturated sodium chloride solution, dried over anhydrous sodium sulfate, filtered and evaporated to dryness at reduced pressure. The residue is purified by preparative thin layer chromatography on silica gel plates utilizing 4:1 petroleum ether/acetone as the solvent to obtain the product as a pale yellow oil.

The product is a racemate that may be resolved by conventional means to obtain the 5R and 5S enantiomers.

*Step 8* (Reaction a))
Ethyl *erythro*-(E)-3,5-dihydroxy-7-[3'-(4''-fluorophenyl)spiro[cyclopentane-1,1'(1H)-inden]-2'-yl]hept-5-enoate (Compound No. 1)

(a) 0.8 ml of 1M. triethylborane/tetrahydrofuran (0.8 mmole) is added to a solution of 300 mg (0.67 mmole) of Compound IVa in 10 ml of dry tetrahydrofuran stirred at 20°—25°C, 0.2 ml of air is added *via* syringe, the reaction mixture is stirred at 20°—25°C for 2 hours and cooled to −78°C, 0.06 g (1.59 mmoles) of sodium borohydride is added in one portion, and the reaction mixture is stirred at −78°C for 48 hours, the reaction mixture being maintained under nitrogen throughout. The cooling bath is removed, and 1N. hydrochloric acid is slowly added dropwise until the evolution of hydrogen ceases and the mixture is acidic (pH~5), the internal temperature of the mixture being maintained below −20°C throughout. 10 ml of water is added, the mixture is extracted three times with diethyl ether, and the diethyl ether extracts are combined, washed twice with water, washed with saturated sodium chloride solution, dried over anhydrous sodium sulfate, filtered and evaporated at reduced pressure to obtain a crude oil.

(b) A solution of the product of Part (a) in 5 ml of methanol is stirred at 20°—25°C for 66 hours under nitrogen and evaporated to dryness at reduced pressure. The residue is chromatographed on a silica gel column utilizing 1:1 diethyl ether/petroleum ether as the eluant to obtain the crude product which solidifies on standing. Repeated recrystallisation from diethyl ether/hexane give the product as a white solid, m.p. 90°—93°C.

N.M.R. ($CDCl_3$): 1.3 (t, 3H), 1.6—1.9 (m, 4H), 2.2 (m, 6H), 2.5 (m, 2H), 3.2 (bs, 1H), 3.7 (bs, 1H), 4.15 (q, 2H), 4.25 (m, 1H), 4.45 (m, 1H), 5.8 (dd, ($J_1$=8 Hz., $J_2$=20 Hz.), 1H), 6.5 (d (J=20 Hz.) 1H), 7.0—7.5 (m, 8H).

Example 2

Sodium *erythro*-(E)-3,5-dihydroxy-7-[3'-(4''-fluorophenyl)-spiro[cyclopentane-1,1'(1H)-inden]-2'-yl]hept-6-enoate (Compound no. 2)

0.11 ml of 1N. sodium hydroxide solution (0.11 mmole) is added to a solution of 50 mg (0.111 mmole) of Compound No. 1 in 3 ml of absolute ethanol stirred at 0°C, and the reaction mixture is stirred at 0°C under nitrogen for 1.5 hours and evaporated to dryness at reduced pressure. The residue is washed three times with diethyl ether to obtain the product, m.p. > 170°C (dec.).

N.M.R. ($CDCl_3$ + $CD_3OD$): 1.5—2.5 (m, 12H), 14.1 (m, 1H), 4.3 (m, 1H), 5.8 (dd ($J_1$=8 Hz., $J_2$=20 Hz.), 1H), 6.4 (d (J=20 Hz.), 1H), 7.0—7.5 (m, 8H).

Compounds 1 and 2 are about 24:1 mixtures of the *erythro* and *threo* racemates which may be separated by conventional means. The principal product, the *erythro* racemate, may be resolved into two optically pure enantiomers, the 3R,5S and 3S,5R isomers, of which the former is preferred. The minor product, the *threo* racemate, may be resolved into the 3R,5R and 3S,5S isomers, of which the former is preferred. The use of a starting material synthesized by utilizing a non-stereoselective reduction would afford a mixture of all four stereoisomers wherein the ratio of the *erythro* isomers to the *threo* isomers ranges from 3:2 to 2:3.

Example 3

(E)-3,5-dihydroxy-7-[3'-(3'',5''-dimethylphenyl)spiro[cyclopentane-1,1'-(1H)-inden]-2'-yl]hept-6-enoic acid, its sodium salt and its ethyl ester (Compounds Nos. 3, 4 and 5)

(a) 31 ml of 1M. triethylborane/tetrahydrofuran (31 mmoles) is added to a solution of 12.0 g (≤26.2 mmoles) of ethyl (E)-7-[3'-(3'',5''-dimethylphenyl)spiro[cyclopentane-1,1'-(1H)-inden]-2'-yl]-5-hydroxy-3-oxo-hept-6-enoate (Compound IVb) (preparable analogously to Example 1 steps 1 to 7) in 500 ml of dry tetrahydrofuran stirred at 20°—25°C, 50 ml of air (at 25°C and 760 mm Hg.) is added *via* a syringe, the reaction mixture is stirred at 20°—25°C for 2 hours and cooled to −78°C, 1.14 g (30.2 mmoles) of sodium borohydride is added in one portion, and the reaction mixture is stirred for 16 hours at −78°C and allowed to warm to 20°—25°C, the reaction mixture being maintained under nitrogen throughout. The reaction mixture is evaporated to dryness at reduced pressure, the residue is vacuum dried, diethyl ether is added, the insoluble material is removed by filtration, and the filtrate is evaporated at reduced pressure. 50 ml of water is added to the oily residue, and the mixture is extracted twice with diethyl ether. The diethyl ether extracts are combined and cooled to 0°C, 10 ml of methanol, 5 ml of 30% aqueous hydrogen peroxide and 10 ml of an aqueous phosphate buffer having a pH of 7 (0.054M. sodium, 0.024M. potassium and 0.047M. phosphate) are added, and the reaction mixture is stirred at 0°C under nitrogen for 45 minutes. Most of the diethyl ether and methanol is evaporated at reduced pressure, the residual aqueous solution is extracted with diethyl ether three times, and the diethyl ether extracts are combined and evaporated at reduced pressure to obtain the crude product as a yellow oil (Compound No. 5).

N.M.R. ($CDCl_3$): 1.25 (t, 3H), 1.6—2.3 (m, 10H), 2.4 (s, 6H), 2.5 (m, 2H), 3.5 (s, 1H), 3.7 (d, 1H), 4.15 (q, 2H), 4.25 (m, 1H), 4.4 (m, 1H), 5.7 (dd, 1H), 6.5 (d (J=20 Hz.), 1H), 6.95—7.4 (m, 7H).

(b) The aqueous layer from the initial diethyl ether extraction (prior to the addition of methanol, hydrogen peroxide and buffer) is acidified with dilute hydrochloric acid, and the mixture is extracted twice with ethyl acetate. The ethyl acetate extracts are combined, dried over anhydrous sodium sulfate, filtered and evaporated at reduced pressure to obtain the crude compound as a foam (Compound No. 3).

(c) 6.5 ml of 1N. sodium hydroxide solution (6.5 mmoles) is added to a solution of 3 g (≤6.5 mmoles) of trhe crude compound (from Part (a)) in 25 ml of ethanol stirred at 0°, and the reaction mixture is stirred at 0°C under nitrogen for 30 minutes, washed with diethyl ether, acidified with dilute hydrochloric acid and extracted with diethyl ether twice. The diethyl ether extracts are combined, dried over anhydrous sodium sulfate, filtered and evaporated at reduced pressure to obtain Compound No. 3 as an oil.

The reaction mixture, prior to the acidification, contains the sodium salt of Compound No. 3 (Compound No. 4). It may be isolated and purified conventionally. m.p. >160°C (dec.).

N.M.R. ($CDCl_3$ + $CD_4OD$): 1.5—2.35 (m, 12H), 2.3 (s, 6H), 4.1 (m, 1H), 4.3 (m, 1H), 5.75 (dd, 1H), 6.45 (d (H= 20 Hz.), 1H), 6.95—7.4 (m, 7H).

Compounds 3, 4 and 5 are approximately 3—9:1 mixtures of the *erythro* and *threo* racemates which may be separated by conventional means, e.g. lactonization of the free acid, separation of the *cis* and *trans* lactones, hydrolysis of the lactones, etc. The principal product, the *erythro* racemate in each case, may be resolved into two optically pure enantiomers, 3R,5S and 3S,5R enantiomers, of which the former is preferred. The minor product, the *threo* racement in each case, may be resolved to obtain the 3R,5R and 3S,5S enantiomers, of which the former is preferred. The use of a non-stereoselective reduction would afford a mixture of all four stereoisomers wherein the ratio of the *erythro* stereoisomers to the *threo* stereoisomers ranges from 3:2 to 2:3.

Example 4

(E)-*Trans*-6-(2'-[3''-(3''',5'''-dimethylphenyl)spiro[cyclopentane-1,1'-(1H)-inden]-2'-yl]ethenyl)-4-hydroxy-3,4,5,6-tetrahydro-2H-pyran-2-one (Compound No. 6) and the corresponding *cis* lactone (Compound No. 7)

(a) 8.7 g (20.5 mmoles) of N-cyclohexyl-N'[2'-(N''-methylmorpholinium)ethyl]carbodiimide *p*-toluenesulphonate is added to a solution of 8.7 g (≥20.1 mmoles) of Compound No. 3 in 250 ml of methylene

chloride (freshly filtered through basic alumina), and the reaction mixture is stirred at 20°—25°C under nitrogen for about 3 hours (until no Compound No. 3 is detectable by thin layer chromatography) and evaporated to dryness at reduced pressure. Water is added, and the mixture is extracted three times with diethyl ether. The diethyl ether extracts are combined, washed with saturated sodium chloride solution, dried over anhydrous sodium sulfate, filtered and evaporated to dryness at reduced pressure to obtain an about 3—4:1 mixture of Compounds No. 6 and No. 7 as a yellow foam.

(b) The product of Part (a) is separated on a Waters Prep-500 high pressure liquid chromatography apparatus utilizing a silica gel column and 15:4.5:10.5 *n*-hexane/acetonitrile/methyl *t*-butyl ether to elute the *trans* lactone (Compound No. 6), a solid foam.

N.M.R. ($CDCl_3$): 1.7—2.3 (m, 10H), 2.35 (s, 6H), 2.7 (m, 2H), 4.4 (m, 1H), 5.25 (m, 1H), 5.75 (dd ($J_1$=10 Hz., $J_2$=20 Hz.), 1H), 6.55 (d (J=20 Hz.), 1H), 6.9—7.5 (m, 7H).

Also eluted from the column is the *cis* lactone (Compound No. 7), also a solid foam.

N.M.R. ($CDCl_3$): 1.7—2.5 (m, 10H), 2.35 (s, 6H), 2.8 (m, 2H), 4.3 (m, 1H), 4.7 (m, 1H), 5.75 (dd ($J_1$=10 Hz., $J_2$=20 Hz.), 1H), 6.5 (d (J=20 Hz) Hz.), 1H), 6.95—7.4 (m, 7H).

Compounds No. 6 and No. 7 are both racemates that may be resolved by conventional means to obtain, in the case of the former, the 4R,6S and 4S,6R enantiomers, of which the former is preferred, and, in the case of the latter, the 4R,6R and 4S,6S enantiomers, of which the former is preferred.

Example 5

Sodium *erythro*-(E)-3,5-dihydroxy-7-[3'-(3'',5''-dimethylphenyl)-spiro[cyclopentane-1,1'(1H)-inden]-2'-yl]hept-6-enoate (Compound No. 8) and Sodium *threo*-(E)-3,5-dihydroxy-7-[3'-(3'',5''-dimethylphenyl)-spiro[cyclopentane-1,1'(1H)-inden]-2'-yl]hept-6-enoate (Compound No. 9)

0.16 of 1N. sodium hydroxide solution (0.16 mmole) is added to a solution of 70 mg (0.169 mmole) of Compound No. 6 in 3 ml of absolute ethanol stirred at 0°C, and the reaction mixture is stirred at 0°C under nitrogen for 30 mintues and evaporated to dryness at reduced pressure. The residue is washed with anhydrous diethyl ether and vacuum dried to obtain the product as a pale yellow solid m.p. >170°C (dec.)

N.M.R. ($CDCl_3$ + $CD_3OD$): 1.5—2.35 (m, 12H), 2.3 (s, 6H), 4.1 (bs, 1H), 4.3 (bs, 1H), 5.75 (dd ($J_1$=10 Hz., $J_2$=20 Hz.), 1H), 6.45 (d (J=20 Hz.), 1H, 6.95—7.4 (m, 7H).

Compound No. 9 is prepared analogously from Compound No. 7. m.p. 160°C (dec.)

N.M.R. ($CDCl_3$ + $CD_3OD$): Essentially the same as that of Compound No. 8.

Compounds Nos. 8 and 9 are racemates that may be resolved by conventional means to obtain 3R,5S and 3S,5R enantiomers (no. 8) and 3R,5R and 3S,5S (No. 9), of which the former in each case are preferred.

Example 6

Ethyl (±)-(E)-7-[3'-(4''-fluorophenyl)-spiro[cyclopentane-1,1'(1H)-inden]-2'-yl]-3-hydroxy-5-oxohept-6-enoate (Compound No. 10)

A mixture of 310 mg of Compound No. 1, 600 mg of activated manganese dioxide and 5 ml of toluene is stirred under nitrogen at 20°—25°C for 24 hours, at 60°C for 8 hours, at 20°—25°C for 16 hours at 80°C for 8 hours and allowed to cool to 20°—25°C. Diethyl ether is added, the mixture is filtered and the filtrate is evaporated at reduced pressure to obtain an oil. The oil is purified by preparative thin layer chromatography on silica gel plate utilizing 80% diethyl ether/petroleum ether as the solvent. The band containing the product is scraped and eluted with ethyl acetate and the solution is filtered and evaporated at reduced pressure to obtain the product as a yellow solid, m.p. 107°—109°C.

The product is a racemate that may be resolved by conventional means to obtain the 3R and 3S enantiomers.

Example 7

Ethyl (±)-(E)-5,5-dimethoxy-7-[3'-(4''-fluorophenyl)-spiro[cyclopentane-1,1'(1H)-inden]-2'-yl]-3-hydroxyhept-6-enoate (Compound No. 11)

A mixture of 90 mg of Compound No. 10, 0.1 ml of trimethyl orthoformate, 2 mg of pyridinium p-toluenesulfonate and 3 ml of methylene chloride is stirred under nitrogen for 45 hours at 20°—25°C and evaporated at reduced pressure, and the residual oil is purified by preparative thin layer chromatography on a silica gel plate utilizing 60% diethyl ether/petroleum ether as the solvent. The band containing the product is scraped and eluted with ethyl acetate and the solution is filtered and evaporated at reduced pressure to obtain the product as a yellow oil.

The product is a racemate that may be resolved by conventional means to obtain the 3R and 3S enantiomers.

The following compounds may be prepared analogously or as otherwise described hereinbefore.

TABLE I

Compounds of Group IAa ( wherein Ro is ring A)

| Cmpd. No. | R | $R_1$ | $R_2$, $R_3$ | $R_4$,$R_5$,$R_6$ | X | $R_{10}$ | $R_{11}$ | Isomers | m.p. |
|---|---|---|---|---|---|---|---|---|---|
| 12 | H | i-$C_3H_7$ | H | 4-F | (E)-CH=CH- | H | $C_2H_5$ | Dl; E:T = ~ 85:15 | Oil |
| 13 | H | i-$C_3H_7$ | | | (E)-CH=CH- | H | Na | Dl; E | > 190°C (dec.) |
| 14 | $CH_3$ | $CH_3$ | | | (E)-CH=CH- | H | $C_2H_5$ | E:T = ~ 9:1 | Oil |
| 15 | $CH_3$ | $CH_3$ | | | (E)-CH=CH- | H | Na | E | > 160°C (dec.) |
| 16 | $C_2H_5$ | $C_2H_5$ | | | (E)-CH=CH- | H | $C_2H_5$ | E:T = ~ 4:1 | Oil |
| 17 | $C_2H_5$ | $C_2H_5$ | | ↓ | (E)-CH=CH- | H | Na | E:T = ~ 4:1 | > 170°C (dec.) |
| 18 | $CH_3$ | $CH_3$ | | 3,5di$CH_3$ | (E)-CH=CH- | H | $C_2H_5$ | E:T = ~ 9:1 | Oil |
| 19 | $CH_3$ | $CH_3$ | ↓ | 3,5di$CH_3$ | (E)-CH=CH- | H | Na | E:T = ~ 9:1 | > 190°C (dec.) |

## TABLE II

(Compounds of Group IAb; Ro = ring A)

| Cmpd. No. | R | $R_1$ | $R_2$, $R_3$ | $R_4$,$R_5$,$R_6$ | X | $R_{10}$ | Isomers | m.p. |
|---|---|---|---|---|---|---|---|---|
| 20 | H | i-$C_3H_7$ | H | 4-F | (E)-CH=CH- | H | Dl; trans | Oil |
| 21 | H | i-$C_3H_7$ | ↓ | 4-F | (E)-CH=CH- | H | Dl; cis: trans =~ 4:1 | Oil |
| 22 | $CH_3$ | $CH_3$ | ↓ | 4-F | (E)-CH=CH- | H | trans | 64°-66°C |
| 23 | $CH_3$ | $CH_3$ | ↓ | 3,5diCH$_3$ | (E)-CH=CH- | H | trans : cis = ~ 4:1 | Foam |

# TABLE III

(Compounds of Group IBa; Ro = ring A)

| Cmpd. No. | R + $R_1$ | $R_2$,$R_3$ | $R_4$,$R_5$,$R_6$ | X | $R_{10}$ | $R_{11}$ | Isomers | m.p. |
|---|---|---|---|---|---|---|---|---|
| 1 | $(CH_2)_4$ | H | 4-F | (E)-CH=CH- | H | $C_2H_5$ | E:T = ~24:1 | 90-93° |
| 2 | $(CH_2)_4$ | H | 4-F | (E)-CH=CH- | H | Na | E:T = ~24:1 | > 170° (dec.) |
| 3 | $(CH_2)_4$ | H | 3,5diCH$_3$ | (E)-CH=CH- | H | H | E:T = ~3-9:1 | Oil |
| 4 | $(CH_2)_4$ | H | 3,5diCH$_3$ | (E)-CH=CH- | H | Na | E:T = ~3-9:1 | > 160° (dec.) |
| 5 | $(CH_2)_4$ | H | 3,5diCH$_3$ | (E)-CH=CH- | H | $C_2H_5$ | E:T = ~3-9:1 | Oil |
| 8 | $(CH_2)_4$ | H | 3,5diCH$_3$ | (E)-CH=CH- | H | Na | Erythro | > 170° (dec.) |
| 9 | $(CH_2)_4$ | H | 3,5diCH$_3$ | (E)-CH=CH- | H | Na | Threo | > 160° (dec.) |
| 24 | $(CH_2)_2$ | H | 4-F | (E)-CH=CH- | H | $C_2H_5$ | E:T = ~ 89:11 | Oil |
| 25 | $(CH_2)_2$ | H | 4-F | (E)-CH=CH- | H | Na | E:T = ~ 9:1 | > 160° (dec.) |
| 26 | $(CH_2)_5$ | H | 4-F | (E)-CH=CH- | H | $C_2H_5$ | E:T = ~ 3:1 | Oil |
| 27 | $(CH_2)_4$ | H | H | (E)-CH=CH- | H | $C_2H_5$ | E:T = ~ 3:1 | Oil |
| 28 | $(CH_2)_4$ | H | 4-F | $(CH_2)_2$ | H | $C_2H_5$ | E:T = ~ 9:1 | Oil |

TABLE IV

(Compounds of Group IBb; Ro = ring A)

| Cmpd. No. | R + $R_1$ | $R_2$,$R_3$ | $R_4$,$R_5$,$R_6$ | X | $R_{10}$ | Isomers | m.p. |
|---|---|---|---|---|---|---|---|
| 6 | $(CH_2)_4$ | H | 3,5di$CH_3$ | (E)-CH=CH- | H | trans | Solid foam |
| 7 | $(CH_2)_4$ | H | 3,5di$CH_3$ | (E)-CH=CH- | H | cis | Solid foam |
| 30 | $(CH_2)_4$ | H | 4-F | (E)-CH=CH- | H | trans:cis=~3:1 | Oil |

TABLE V

(Compounds of Group IBa)

| Cmpd. No. | R + $R_1$ | $R_2$,$R_3$ | Ro | X | $R_{10}$ | $R_{11}$ | Isomers | m.p. |
|---|---|---|---|---|---|---|---|---|
| 29 | $(CH_2)_4$ | H | $\underline{i}-C_3H_7$ | (E)-CH=CH- | H | $C_2H_5$ | E:T = ∼9:1 | Oil |

TABLE VI

(Compounds of Group IBc; Ro = ring A)

| Cmpd. No. | R + $R_1$ | $R_2$,$R_3$ | $R_4$,$R_5$,$R_6$ | X | $R_{10}$ | $R_{11}$ | Q | Isomers | m.p. |
|---|---|---|---|---|---|---|---|---|---|
| 10 | $(CH_2)_4$ | H | 4-F | (E)-CH=CH- | H | $C_2H_5$ | 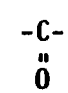 | - | 107-109° |
| 11 | $(CH_2)_4$ | H | 4-F | (E)-CH=CH- | H | $C_2H_5$ | 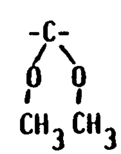 | - | Oil |

In Tables I—VI

D1 = approximately 1:1 mixture of diasteroisomers with respect to the 1-position of the indene ring

E = *erythro* racemate

T = *threo* racemate

*cis* = cis lactone

*trans* = *trans* lactone

Thus, for example, "D1; E:T = ~85:15" means that the compound is a mixture of eight stereoisomers wherein the ratio of the four *erythro* stereoisomers to the four *threo* stereoisomers is about 85:15 and the ratio of the four stereoisomers wherein $R_1$ has one configuration to the four stereoisomers wherein $R_1$ has the opposite configuration is about 1:1.

N.M.R. Data

| Cmpd. No. | | |
|---|---|---|
| 12 | ($CDCl_3$): | 0.3 (d (J=10 Hz.), 3H), 1.2 (t, 3H), 1.35 (d (J=10 Hz.), 3H), 1.7 (m, 2H), 2.5 (m, 2H), 3.3 (s, 1H), 3.7 (m, 1H), 4.2 (q, 2H), 4.3 (m, 1H), 4.5 (m, 1H), 5.8 (dd ($J_1$=10 Hz., $J_2$=20 Hz.), 1H), 6.5 (d (J=20 Hz.), 1H), 7.0—7.5 (m, 8H). |
| 13 | ($CDCl_3$ + $CD_3OD$): | 0.35 (d (J=10 Hz.), 3H), 1.4 (d (J=10 Hz.), 3H), 1.65 (m, 2H), 2.2—2.6 (m, 3H), 3.75 (bs, 1H), 4.15 (m, 1H), 4.4 (m, 1H), 5.9 (dd ($J_1$=10 Hz., $J_2$=20 Hz.), 1H), 6.55 (d (J=20 Hz.), 1H), 7.0—7.5 (m, 8H). |
| 14 | ($CDCl_3$): | 1.3 (t, 3H), 1.5 (d, 6H), 1.6—1.9 (m, 2H), 2.5 (d, 2H), 4.2 (q, 2H), 4.3 (m, 1H), 4.5 (m, 1H), 6.0 (dd ($J_1$=10 Hz., $J_2$=20 Hz.), 1H), 6.55 (d (J=20 Hz.), 1H), 7.1—7.4 (m, 8H). |
| 15 | ($CDCl_3$ + $CD_4OD$): | 1.4 (d, 6H), 1.5 (m, 2H), 2.2 (m, 2H), 4.15 (m, 1H), 4.3 (m, 1H), 5.9 (dd ($J_1$=10 Hz., $J_2$=20 Hz.), 1H), 6.4 (d (J=20 Hz.), 1H), 7.0—7.4 (m, 8H). |
| 16 | ($CDCl_3$): | 0.35 (m, 6H), 1.3 (t (J=10 Hz.), 3H), 1.7 (m, 4H), 2.0 (m, 4H), 2.5 (d (J=10 Hz.), 2H), 4.2 (q (J=10 Hz.), 2H), 4.3 (m, 1H), 4.45 (m, 1H), 5.9 (dd ($J_1$=10 Hz., $J_2$=20 Hz.), 1H), 6.5 (d (J=20 Hz.), 1H), 7.1—7.4 (m, 8H). |
| 17 | ($CDCl_3$ + $CD_3OD$): | 0.35 (m, 6H), 1.7 (m, 2H), 2.0 (m, 4H), 2.3 (m, 2H), 4.1 (m, 1H), 4.35 (m, 1H), 5.9 (dd ($J_1$=10 Hz., $J_2$=20 Hz.), 1H), 6.5 (d (J=20 Hz.), 1 H), 7.0—7.5 (m, 8H). |
| 18 | ($CDCl_3$): | 1.25 (t, 3H), 1.5 (d (J=8 Hz.), 6H), 1.55—1.9 (m, 2H), 2.35 (s, 6H), 2.5 (d, 2H), 4.15 (q, 2H), 4.3 (m, 1H), 4.45 (m, 1H), 5.9 (dd ($J_1$=10 Hz., $J_2$=20 Hz.), 1H), 6.55 (d (J=20 Hz.), 1H), 7.0—7.4 (m, 7H). |
| 19 | ($CDCl_3$ + $CD_4OD$): | 1.4 (d, 6H), 1.5—1.9 (m, 2H), 2.2—2.5 (m, 8H), 4.1 (m, 1H), 4.45 (m, 1H), 5.9 (dd ($J_1$=10 Hz., $J_2$=20 Hz.), 1H), 6.5 (d (J=20 Hz.), 1H), 7.0—7.4 (m, 7H). |
| 20 | ($CDCl_3$): | 0.35 (d (J=10 Hz.), 3H), 1.4 (d (J=10 Hz.), 3H), 1.8—2.2 (m, 3H), 2.4—2.9 (m, 3H), 3.8 (bs, 1H), 4.45 (bs, 1H), 5.3 (m, 1H), 5.9 (dq, 1H), 6.6 (d (J=20 Hz.), 1H), 7.0—7.6 (m, 8H). |
| 21 | ($CDCl_3$): | 0.35 (d (J=10 Hz.), 3H), 1.4 (d (J=10 Hz.), 3H), 1.8—2.2 (m, 2H), 2.25—3.05 (m, 4H), 3.8 (bs, 1H), 4.3 (m, 1H), 4.8 (m, 1H), 5.9 (m, 1H), 6.6 (d (J=20 Hz.), 1H), 7.0—7.6 (m, 8H). |
| 22 | ($CDCl_3$): | 1.5 (d (J=8 Hz.), 6H), 1.8—2.1 (m, 2H), 2.5—2.8 (m, 2H), 4.4 (m, 1H), 5.2 (m, 1H), 5.9 (dd ($J_1$=10 Hz., $J_2$=20 Hz.), 1H), 6.55 (d (J=20 Hz.), 1H), 7.1 —7.5 (m, 8H). |
| 23 | ($CDCl_3$): | 1.5 (d, 6H), 1.8—2.1 (m, 2H), 2.4 (s, 6H), 2.5—3.0 (m, 2H), 4.4 (m, 1H), 5.2 (m, 1H), 5.9 (dd, 1H), 6.6 (d, 1H), 6.95—7.45 (m, 7H). |

N.M.R. Data

| Cmpd. No. | | |
|---|---|---|
| 24 | ($CDCl_3$): | 1.3 (t, 3H), 1.5—2.0 (m, 6H), 2.5 (d, 2H), 3.2 (s, 1H), 3.8 (s, 1H), 4.2 (q, 2H), 4.2—4.45 (m, 2H), 5.5 (dd, ($J_1$=10 Hz., $J_2$=20 Hz.), 1H), 6.5 (d (J=20 Hz.), 1H), 7.0—7.45 (m, 8H). |
| 25 | ($CDCl_3$ + $CD_3OD$): | 1.5—2.4 (m, 8H), 4.1 (m, 1H), 4.3 (m, 1H), 5.5 (dd ($J_1$=10 Hz., $J_2$=20 Hz.), 1H), 6.4 (d (J=20 Hz.), 1H), 7.0—7.5 (m, 8H). |
| 26 | ($C_6D_6$) | 0.9 (t, J=10 Hz, 3H), 1.1—2.4 (m, 14H), 3.8 (q, J=10 Hz, 2H), 3.9—4.4 (m, 2H), 6.0 (dd, J=20 and 10 Hz, 1H), 6.7 (m, 1H), 6.8—7.8 (m, 8H). |
| 27 | ($C_6D_6$) | 0.9 (m, 2H), 1.2—2.5 (m, 12H), 3.8 (m, 2H), 4.0 (m, 1H), 4.1—4.3 (m, 1H), 5.9 (dd, J=20 and 10 Hz, 1H), 6.8 (dd, J=20 and 10 Hz, 1H), 6.9—7.5 (m, 9H). |
| 11 | ($C_6D_6$) | 0.9 (t, J=10 Hz, 3H), 1.2—3.1 (m, 12H), 3.2 (ds, 6H), 3.9 (q, J=10 Hz, 2H), 4.8 (m, 1H), 6.5 (d, J=20 Hz, 1H), 6.8—7.4 (m, 8H), 7.7 (d, J=20 Hz, 1H). |
| 28 | ($CDCl_3$): | 1.3 (t, 3H), 1.4—2.5 (m, 16H), 3.7 (m, 2H), 4.2 (q, J=10 Hz, 2H), 6.8—7.3 (m, 8H). |
| 30 | ($CDCl_3$): | 1.8—3.0 (m, 12H), 4.4 (m, 1H), 5.2 (m, 1H), 5.7 (dd, J=10 and 20 Hz, 1H), 6.5 (d, J=20 Hz, 1H), 7.1—7.5 (m, 8H). The following additional smaller peaks: 4.3 (m), 4.7 (m), 5.8 (dd), 6.45 (d), due to the corresponding *cis* lactone. |
| 29 | ($C_6D_6$): | 0.9 (t, J=10 Hz, 3H), 1.4 (dd, J=10 and 20 Hz, 6H), 1.5—2.4 (m, 12H), 3.4 (m, 1H), 3.8 (q, J=10 Hz, 2H), 4.15 (m, 1H), 4.4 (m, 1H), 5.8 (dd, J=20 and 10 Hz, 1H), 6.9 (dd, J=20 and 1 Hz, 1H), 7.0—7.6 (m, 4H). The following small peaks at 4.55 (m), 5.9 (dd), 6.8 (d), due to the *threo* isomer. |

All nuclear magnetic resonance spectra were taken at ambient temperature on a 200 MHz. spectrometer. All chemical shifts are given in p.p.m. (δ) relative to tetramethylsilane, and where a single δ value is given for anything other than a sharp singlet, it is its center point. In the N.M.R. data:

bs = broad singlet
d = doublet
dd = doublet of a doublet
dq = doublet of a quartet
m = multiplet
q = quartet
s = singlet
t = triplet
ds = double singlet

**Claims for the Contrating States: BE CH FR GB IT LI LU NL SE DE**

1. A compound of the formula I

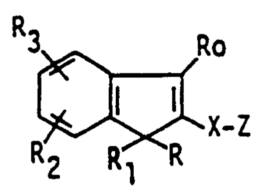

I

wherein
R is hydrogen or primary or secondary $C_{1-6}$alkyl,
$R_1$ is primary or secondary $C_{1-6}$alkyl or

R and $R_1$ together are $(CH_2)_m$ or (Z)—$CH_2$—CH=CH—$CH_2$— wherein m is 2, 3, 4, 5 or 6,
Ro is $C_{1-6}$alkyl, $C_{3-7}$cycloalkyl or ring A

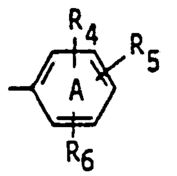

each of $R_2$ and $R_4$ is independently hydrogen, $C_{1-4}$alkyl, $C_{1-4}$alkoxy (except t-butoxy), trifluoromethyl, fluoro, chloro, phenoxy or benzyloxy,

each of $R_3$ and $R_5$ is independently hydrogen, $C_{1-3}$alkyl, $C_{1-3}$alkoxy, trifluoromethyl, fluoro, chloro, phenoxy or benzyloxy,

$R_6$ is hydrogen, $C_{1-2}$alkyl, $C_{1-2}$alkoxy, fluoro, or chloro,
with the proviso that there may be one each of trifluoromethyl, phenoxy or benzyloxy on each of the phenyl and indene rings

X is $—(CH_2)_n—$ or $—(CH_2)_qCH=CH(CH_2)_q—$
wherein n is 1, 2 or 3 and both q's are 0 or one is 0 and the other is 1,

and

Z is 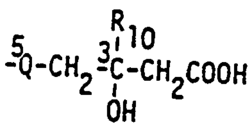 II

wherein

Q is 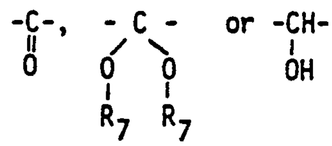

, or

wherein each $R_7$ is the same primary or secondary $C_{1-6}$alkyl or together they represent $—(CH_2)_2—$, $—(CH_2)_3—$,

$R_{10}$ is hydrogen or $C_{1-3}$alkyl,
with the provision that Q may be other than

—CH—
 |
OH

only when X is —CH=CH— or —$CH_2$—CH=CH— and/or $R_{10}$ is $C_{1-3}$alkyl, in free acid form, or in the form of an ester or δ-lactone thereof or in salt form as appropriate.

2. A compound according to Claim 1 wherein Ro represents ring A

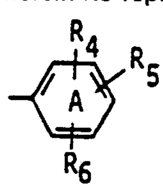

R is hydrogen or primary or secondary $C_{1-6}$alkyl not containing an asymmetric carbon atom, and

$R_1$ is primary or secondary $C_{1-6}$alkyl not containing an asymmetric carbon atom or

R and $R_1$ taken together are $—(CH_2)_m—$ or (Z)—$CH_2$CH=CH—$CH_2$—, wherein m is 2, 3, 4, 5 or 6,

$R_2$ is hydrogen, $C_{1-3}$alkyl, *n*-butyl, *i*-butyl, *t*-butyl, $C_{1-3}$alkoxy, *n*-butoxy, *i*-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy,
with the proviso that not more than one of $R_2$ and $R_3$ is trifluoromethyl,
not more than one of $R_2$ and $R_3$ is phenoxy,
and not more than one of $R_2$ and $R_3$ is benzyloxy,

$R_4$ is hydrogen, $C_{1-3}$alkyl, *n*-butyl, *i*-butyl, *t*-butyl, $C_{1-3}$alkoxy, *n*-butoxy, *i*-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_6$ is hydrogen, $C_{1-2}$alkyl, $C_{1-2}$alkoxy, fluoro or chloro,

with the provisos that not more than one of $R_4$ and $R_5$ is trifluoromethyl, not more than one of $R_4$ and $R_5$ is phenoxy, and not more than one of $R_4$ and $R_5$ is benzyloxy,

X is $-(CH_2)_n-$ or (E)—CH=CH—,

wherein n is 1, 2 or 3, and

Z 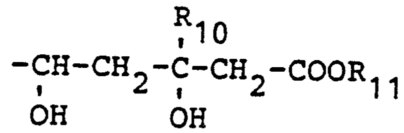 II(a) or 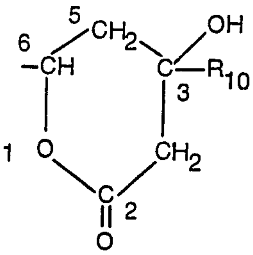 II(b)

wherein $R_{10}$ is hydrogen or $C_{1-3}$alkyl, and

$R_{11}$ is hydrogen, $R_{12}$ or M,

wherein $R_{12}$ is a physiologically acceptable and hydrolyzable ester group, and

M is a pharmaceutically acceptable cation.

3. A compound according to Claim 1, wherein $R_o$ is 4-fluorophenyl.

4. A compound selected from *erythro*-(E)-3,5-dihydroxy-7-[3'-(4''-fluorophenyl)-spiro[cyclopentane-1,1'(1H)-inden]-2'-yl]-hept-6-enoic acid and *erythro*-(E)-3,5-dihydroxy-7-[3'-(3'',5''-dimethylphenyl)-spiro[cyclopentane-1,1'(1H)-inden]-2'-yl]-hept-6-enoic acid in free acid or salt form.

5. A compound according to Claim 4 in sodium salt form.

6. A compound according to Claim 1 which is *erythro*-(E)-3,5-dihydroxy-7-[3'-(4''-fluorophenyl)-spiro[cyclopentane-1,1'(1H)-inden]-2'-yl]-hept-6-enoate in free acid or salt form.

7. A compound according to Claim 1 which is the (3R,5S) enantiomer of a compound according to Claim 6, in free acid or salt form.

8. A compound according to Claim 6 or 7 in sodium salt form.

9. A pharmaceutical composition comprising a compound according to Claim 1 as appropriate in free acid form or in the form of a physiologically hydrolysable and -acceptable ester or a lactone thereof or in pharmaceutically acceptable salt form, together with a pharmaceutically acceptable diluent or carrier.

10. A compound according to Claim 1 as appropriate in free acid form or in the form of a physiologically-hydrolysable and -acceptable ester or a lactone thereof or in pharmaceutically acceptable salt form for use as a pharmaceutical.

11. A compound according to Claim 1 as appropriate in free acid form or in the form of a physiologically-hydrolysable and -acceptable ester or a lactone thereof or in pharmaceutically acceptable salt form for use in inhibiting cholesterol biosynthesis or treating atherosclerosis.

12. A process for preparing a compound according to Claim 1 which comprises hydrolysing a compound of formula I in ester or lactone form or esterifying or lactonising a compound of formula I in free acid form and when a free carboxyl group is present recovering the compound obtained in free acid form or in the form of a salt.

13. A process for preparing a compound according to Claim 1 which comprises

a) when X is $(CH_2)_n$ or (E)—CH=CH— and $R_{10}$ is hydrogen reducing a compound of formula IV

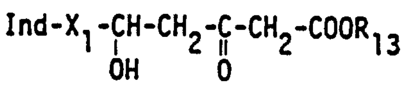 IV

wherein Ind stands for

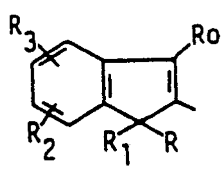

and substituents for Ind are as defined in Claim 1 and wherein $R_{13}$ is a radical forming an ester, and $X_1$ is $(CH_2)_n$ or (E)—CH=CH—,

b) when X is $(CH_2)_n$ or (E)—CH=CH— and $R_{10}$ is $C_{1-3}$alkyl hydrolysing a compound of formula XII

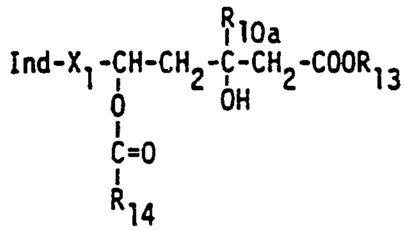

XII

wherein $R_{10a}$ is $C_{1-3}$alkyl, $R_4$ is part of an ester forming group and Ind, $X_1$ and $R_{13}$ are as defined above,

c) when X is —CH=CH— or —$CH_2$—CH=CH— and the group 2 is in the form of a δ-lactone IIb, in 4R,6S configuration

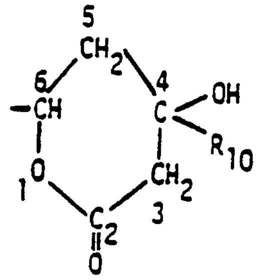

IIb

wherein $R_{10}$ is as defined in claim 1 or

X is —$CH_2CH_2$ or $CH_2CH_2CH_2$ and IIb is in 4R,6R configuration deprotecting a compound of formula XXXIX

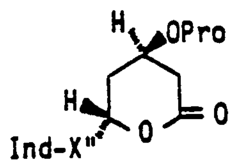

XXXIX

wherein X'' represents —$CH_2CH_2$, —$CH_2CH_2CH_2$, —CH=CH— or —$CH_2$CH=CH— and Pro is a protecting group, and Ind is as defined above,

d) when X is —$(CH_2)_2$—, —$(CH_2)_3$—, —$(CH_2)_q$CH=CH$(CH_2)_q$— deprotecting a compound of formula XXXII

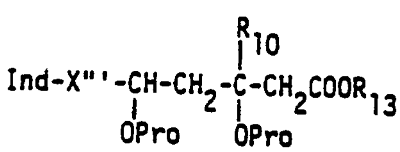

XXXII

wherein X''' is —$(CH_2)_2$—, —$(CH_2)_3$— or —$(CH_2)_q$—CH=CH—$(CH_2)_q$—, and Ind, q, $R_{10}$, $R_{13}$ and Pro are as defined above,

e) when Q is

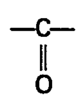

oxidising the corresponding compound of formula I wherein Q is

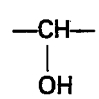

f) when

Q is 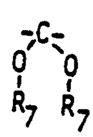

and II is in ester form ketalising the corresponding compound of formula I where Q is

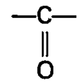

g) hydrolysing a compound of formula I in the form of an ester or a lactone or

h) esterifying or lactonising a compound of formula I in free acid form, and when a free carboxyl group is present, recovering the compound obtained in free acid form or in the form of a salt.

14. A compound of formula IV

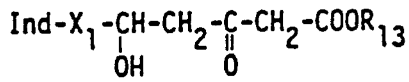

IV

wherein $R_{13}$ is a radical forming an ester, $X_1$ is $(CH_2)_n$ or (E)—CH=CH— and Ind is as defined in claim 13; of formula XII

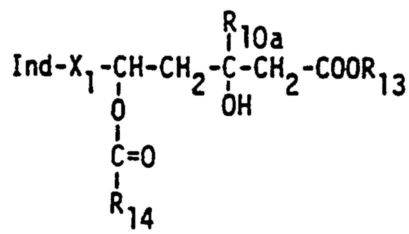

XII

wherein $R_{10a}$ is $C_{1-3}$alkyl, $R_{14}$ is part of an ester forming group and Ind, $X_1$ and $R_{13}$ are as defined above; of formula XXXII

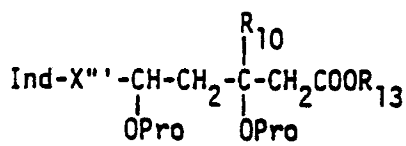

XXXII

wherein X''' is $-(CH_2)_2-$, $-(CH_2)_3$ or $-(CH_2)_q-CH=CH-(CH_2)_q-$ and Ind, q, $R_{10}$, $R_{13}$ and Pro are as defined above; or a formula XXXIX

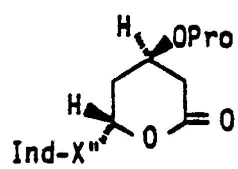

XXXIX

wherein X'' represents $-CH_2CH_2-$, $-CH_2CH_2CH_2-$, —CH=CH— or $-CH_2CH=CH-$, Pro is a protecting group and Ind is as defined above.

**Claims for the Contracting State: AT**

1. A process for the preparation of a compound of formula I

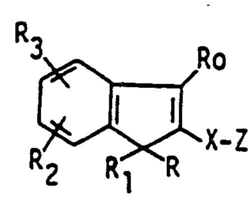

I

wherein

R is hydrogen or primary or secondary $C_{1-6}$alkyl,
$R_1$ is primary or secondary $C_{1-6}$alkyl or

R and $R_1$ together are $(CH_2)_m$ or (Z)—$CH_2$—CH=CH—$CH_2$— wherein m is 2, 3, 4, 5 or 6, Ro is $C_{1-6}$alkyl, $C_{3-7}$cycloalkyl or ring A

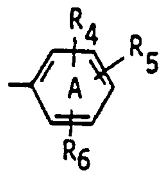

each of $R_2$ and $R_4$ is independently hydrogen, $C_{1-4}$alkyl, $C_{1-4}$alkoxy (except t-butoxy), trifluoromethyl, fluoro, chloro, phenoxy or benzyloxy,

each of $R_3$ and $R_5$ is independently hydrogen, $C_{1-3}$alkyl, $C_{1-3}$alkoxy, trifluoromethyl, fluoro, chloro, phenoxy or benzyloxy,

$R_6$ is hydrogen, $C_{1-2}$alkyl, $C_{1-2}$alkoxy, fluoro, or chloro,

with the proviso that there may be one each of trifluoromethyl, phenoxy or benzyloxy on each of the phenyl and indene rings

X is —$(CH_2)_n$— or —$(CH_2)_q$CH=CH$(CH_2)_q$—

wherein n is 1, 2 or 3 and both q's are 0 or one is 0 and the other is 1,

and

Z is 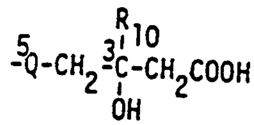 II

wherein

Q is 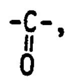, 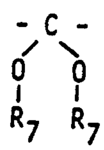 or 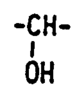

wherein each $R_7$ is the same primary or secondary $C_{1-6}$alkyl or together they represent —$(CH_2)_2$—, —$(CH_2)_3$—,

$R_{10}$ is hydrogen or $C_{1-3}$alkyl,

with the proviso that Q may be other than

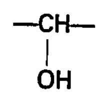

only when X is —CH=CH— or —$CH_2$—CH=CH— and/or $R_{10}$ is $C_{1-3}$alkyl, in free acid form, or in the form of an ester or δ-lactone thereof or in salt form as appropriate, which comprises

a) when X is $(CH_2)_n$ or (E)—CH=CH— and $R_{10}$ is hydrogen reducing a compound of formula IV

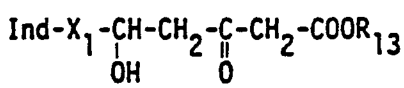 IV

wherein Ind stands for

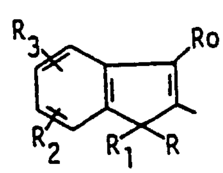

and substituents for Ind are as defined above, and wherein $R_{13}$ is a radical forming an ester, and $X_1$ is $(CH_2)_n$ or (E)—CH=CH—,

b) when X is $(CH_2)_n$ or (E)—CH=CH— and $R_{10}$ is $C_{1-3}$alkyl hydrolysing a compound of formula XII

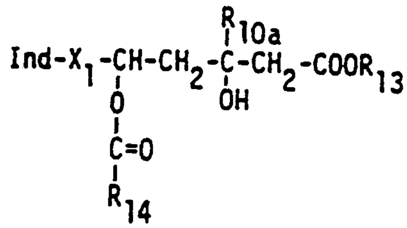

XII

wherein $R_{10a}$ is $C_{1-3}$alkyl, $R_{14}$ is part of an ester forming group and Ind, $X_1$ and $R_{13}$ are as defined above,

c) when X is —CH=CH— or —$CH_2$—CH=CH— and the group 2 is in the form of a δ-lactone of formula IIb, in 4R,6S configuration

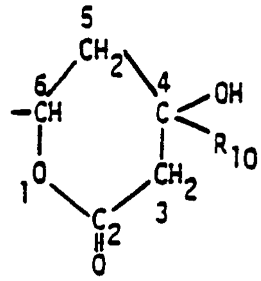

IIb

wherein $R_{10}$ is as defined in claim 1 or

X is —$CH_2CH_2$ or $CH_2CH_2CH_2$ and IIb is in 4R,6R configuration deprotecting a compound of formula XXXIX

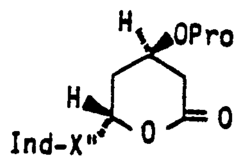

XXXIX

wherein X'' represents —$CH_2CH_2$—, —$CH_2CH_2CH_2$—, —CH=CH— or —$CH_2$CH=CH— and Pro is a protecting group, and Ind is as defined above,

d) when X is —$(CH_2)_2$—, —$(CH_2)_3$—, —$(CH_2)_q$CH=CH$(CH_2)_q$— deprotecting a compound of formula XXXII

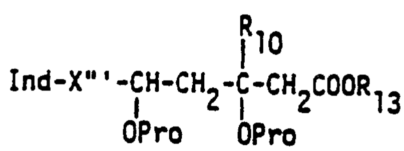

XXXII

wherein X''' is —$(CH_2)_2$—, —$(CH_2)_3$— or —$(CH_2)_q$—CH=CH—$(CH_2)_q$—, and Ind, q, $R_{10}$, $R_{13}$ and Pro are as defined above,

e) when Q is

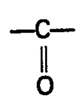

oxidising the corresponding compound of formula I wherein Q is

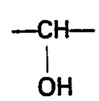

f) when

Q is 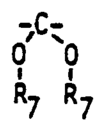

and II is in ester form ketalising the corresponding compound of formula I where Q is

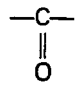

g) hydrolysing a compound of formula I in the form of an ester or a lactone or

h) esterifying or lactonising a compound of formula I in free acid form, and when a free carboxyl group is present, recovering the compound obtained in free acid form or in the form of a salt.

2. A process for the preparation of a compound of formula I as defined in Claim 1 which comprises hydrolysing a compound of formula I in ester or lactone form or esterifying or lactonising a compound of formula I in free acid form and when a free carboxyl group is present recovering the compound obtained in free acid form or in the form of a salt.

3. A process according to claim 1 or 2 for the preparation of a compound of formula I as defined in claim 1 selected from *erythro*-(E)-3,5-dihydroxy-7-[3'-(4''-fluorophenyl)-spiro[cyclopentane-1,1'(1H)-inden]-2'-yl]-hept-6-enoic acid and *erythro*-(E)-3,5-dihydroxy-7-[3'-(3'',5''-dimethylphenyl)-spiro[cyclopentane-1,1'(1H)-inden]-2'-yl]-hept-6-enoic acid in free acid or salt form.

4. A process according to Claim 1 or 2 for the preparation of a compound according to Claim 3 in sodium salt form.

5. A process according to Claim 1 or 2 for the preparation of sodium *erythro*-(E)-3,5-dihydroxy-7-[3'-(4''-fluorophenyl)-spiro[cyclopentane-1,1'(1H)-inden]-2'-yl]-hept-6-enoate in free acid or salt form.

6. A process according to Claim 1 or 2 for the preparation of the (3R,5S) enantiomer of the compound according to Claim 5.

7. A process for the preparation of a pharmaceutical composition which comprises mixing a compound of formula I as defined in Claim 1 in free acid form, or in the form of a pharmaceutically acceptable ester or δ-lactone thereof, or in pharmaceutically acceptable salt form as appropriate, with a pharmaceutically acceptable carrier or diluent.

**Patentansprüche für die Vertragsstaaten: BE CH FR GB IT LI LU NL SE DE**

1. Verbindungen der Formel (I)

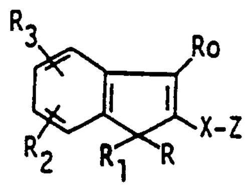

I

worin

R Wasserstoff oder eine primäre oder sekundäre $C_1$—$C_6$-Alkylgruppe,

$R_1$ eine primäre oder sekundäre $C_1$—$C_6$-Alkylgruppe oder

R und $R_1$ zusammen $(CH_2)_m$ oder (Z)—$CH_2$—$CH$=$CH$—$CH_2$— sind, worin m 2, 3, 4, 5 oder 6 ist,

Ro eine $C_1$—$C_6$-Alkyl-, $C_3$—$C_7$-Cycloalkylgruppe oder ein Ring A

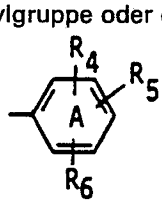

ist,

jeder $R_2$ und $R_4$ unabhängig Wasserstoff, eine $C_1$—$C_4$-Alkyl-, $C_1$—$C_4$-Alkoxy- (mit Aushname t-Butoxy), Trifluoromethyl-, Fluor-, Chlor-, Phenoxy- oder Benzyloxygruppe ist,

jeder $R_3$ und $R_5$ unabhängig Wasserstoff, eine $C_1$—$C_3$-Alkyl-, $C_1$—$C_3$-Alkoxy-, Trifluormethyl-, Fluor-, Chlor-, Phenoxy- oder Benzyloxygruppe ist.

$R_6$ Wasserstoff, eine $C_1$—$C_2$-Alkylgruppe, eine $C_1$—$C_2$-Alkoxygruppe, Fluor oder Chlor ist, mit dem Vorbehalt, daß jeweils nur eine Trifluormethyl-, Phenoxy- oder Benzyloxygruppe an jedem der Phenyl- und Indenringe sein kann,

X —$(CH_2)_n$ oder —$(CH_2)_qCH$=$CH(CH_2)_q$— ist, worin n 1, 2 oder 3 ist und beide q 0 sind oder eines 0 und das andere 1 ist, und

Z

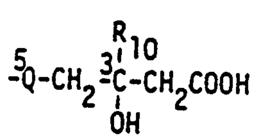

II

ist,

worin

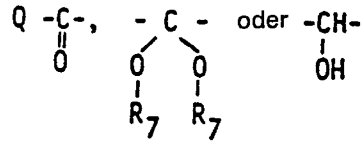

ist, worin jedes $R_7$ die gleiche primäre oder sekundäre $C_1$—$C_6$-Alkylgruppe ist oder zusammen —$(CH_2)_2$—, —$(CH_2)_3$— bilden, $R_{10}$ Wasserstoff oder eine $C_1$—$C_3$-Alkylgruppe ist, mit de Vorbehalt, daß Q etwas anderes als

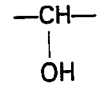

nur sein kann, wenn X —CH=CH— oder —$CH_2$—CH=CH— und/oder $R_{10}$ eine $C_1$—$C_3$-Alkylgruppe ist, in freier Säureform oder in Form eines Esters oder δ-Lactons davon oder in Salzform.

2. Verbindung nach Anspruch 1, worin Ro den Ring A

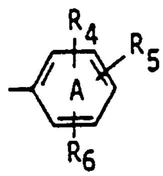

darstellt,

R Wasserstoff oder eine primäre oder sekundäre $C_1$—$C_6$-Alkylgruppe ist, die kein asymmetrisches Kohlenstoffatom enthält, und

$R_1$ eine primäre oder sekundäre $C_1$—$C_6$-Alkylgruppe ist, die kein asymmetrisches Kohlenstoffatom enthält, oder

R und $R_1$ zusammen —$(CH_2)_m$— oder (Z)—$CH_2$—CH=CH—$CH_2$— bilden, worin m 2, 3, 4, 5 oder 6 ist,

$R_2$ Wasserstoff, eine $C_1$—$C_3$-Alkyl-, n-Butyl-, i-Butyl-, t-Butyl-, $C_1$—$C_3$-Alkoxy- n-Butoxy, i-Butoxy-, Trifluormethylgruppe, Fluor, Chlor, Phenoxy- oder Benzyloxygruppe ist,

$R_3$ Wasserstoff, eine $C_1$—$C_3$-Alkyl-, $C_1$—$C_3$-Alkoxy-, Trifluormethylgruppe, Fluor, Chlor, Phenoxy- oder Benzyloxygruppe ist,

mit dem Vorbehalt, daß nicht mehr als einer der Rest $R_2$ und $R_3$ eine Trifluormethylgruppe ist,
nicht mehr als einer der Reste $R_2$ und $R_3$ eine Phenoxygruppe ist,
und nicht mehr als einer der Reste $R_2$ und $R_3$ eine Benzyloxygruppe ist,

$R_4$ Wasserstoff, eine $C_1$—$C_3$-Alkyl-, n-Butyl-, i-Butyl-, t-Butyl-, $C_1$—$C_3$-Alkoxy-, n-Butoxy-, i-Butoxy-, Trifluormethylgruppe, Fluor, Chlor, Phenoxy- oder Benzyloxygruppe ist,

$R_5$ Wasserstoff, eine $C_1$—$C_3$-Alkyl-, $C_1$—$C_3$-Alkoxy-, Trifluormethylgruppe, Fluor, Chlor, Phenoxy- oder Benzyloxygruppe ist,

$R_6$ Wasserstoff, eine $C_1$—$C_2$-Alkyl-, $C_1$—$C_2$-Alkoxygruppe, Fluor oder Chlor ist,

mit dem Vorbehalt, daß nicht mehr als einer der Reste $R_4$ und $R_5$ eine Trifluormethylgruppe ist, nicht mehr als einer der Reste $R_4$ und $R_5$ eine Phenoxygruppe ist und nicht mehr als einer der Reste $R_4$ und $R_5$ eine Benzyloxygruppe ist,

X —$(CH_2)_n$— oder (E)—CH=CH— ist, worin n 1, 2 oder 3 ist, und

Z 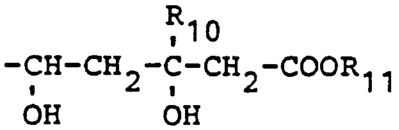 II(a) oder 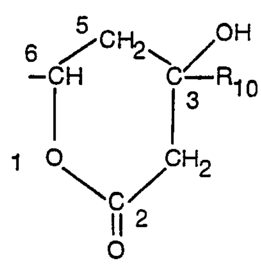 II(b)

ist, worin $R_{10}$ Wasserstoff oder eine $C_1$—$C_3$-Alkylgruppe ist und

$R_{11}$ Wasserstoff, $R_{12}$ oder M ist, worin

$R_{12}$ eine physiologisch annehmbare und hydrolysierbare Estergruppe ist und

M ein pharmazeutisch annehmbares Kation ist.

3. Verbindung nach Anspruch 1, worin Ro für 4-Fluorphenyl steht.

4. Verbindung, ausgewählt *erythro*-(E)-3,5-Dihydroxy-7-[3'-(4''-fluorphenyl)-spiro[cyclopentan-1,1'(1H)-inden]-2'-yl]-hept-6-encarbonsäure und *erythro*-(E)-3,5-Dihydroxy-7-[3'-(3'',5''-dimethylphenyl)-spiro[cyclopentan-1,1'(1H)-inden]-2'-yl]-hept-6-encarbonsäure in Form der freien Säure oder in Form eines Salzes.

5. Verbindung nach Anspruch 4 in Form eines Natriumsalzes.

6. Verbindung nach Anspruch 1, bei der es sich um *erythro*-(E)-3,5-Dihydroxy-7-[3'-(4''-fluorphenyl)-spiro[cyclopentan-1,1'(1H)-inden]-2'-yl]-hept-6-enoat in Form der freien Säure oder in Form eines Salzes handelt.

7. Verbindung nach Anspruch 1, bei der es sich um das (3R,5S)-Enantiomer einer Verbindung nach Anspruch 6 handelt, in Form der freien Säure oder in Form eines Salzes.

8. Verbindung nach Anspruch 6 oder 7 in Form eines Natriumsalzes.

9. Pharmazeutische Zusammensetzung aus einer Verbindung nach Anspruch 1 in Form der freien Säure oder in Form eines physiologisch hydrolysierbaren und physiologisch annehmbaren Esters oder eines Lactons hiervon oder in Form eines pharmazeutisch annehmbaren Salzes zusammen mit einem pharmazeutisch annehmbaren Verdünnungsmittel oder Träger.

10. Verbindung nach Anspruch 1 in Form der freien Säure oder in Form eines physiologisch hydrolysierbaren und physiologisch annehmbaren Esters oder eines Lactons hiervon oder in Form eines pharmazeutisch annehmbaren Salzes zur Anwendung als Pharmazeutikum.

11. Verbindung nach Anspruch 1 in Form der freien Säure oder in Form eines physiologisch hydrolysierbaren und physiologisch annehmbaren Esters oder eines Lactons hiervon oder in Form eines pharmazeutisch annehmbaren Salzes zur Anwendung für die Hemmung der Biosynthese von Chloesterin oder die Behandlung von Atherosklerose.

12. Verfahren zur Herstellung einer Verbindung nach Anspruch 1, dadurch gekennzeichnet, daß eine Verbindung der Formel (I) in Form eines Esters oder Lactons hydrolysiert oder eine Verbindung der Formel (I) in Form der freien Säure verestert oder lactonisiert wird und, falls eine freie Carboxylgruppe vorhanden ist, die erhaltene Verbindung in Form der freien Säure oder in Form eines Salzes gewonnen wird.

13. Verfahren zur Herstellung einer Verbindung nach Anspruch 1, gekennzeichnet,

a) wenn X $(CH_2)_n$ oder (E)—CH=CH— ist und $R_{10}$ Wasserstoff ist, durch Reduzieren einer Verbindung der Formel (IV)

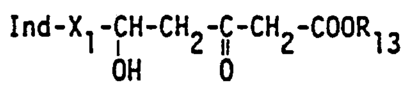

IV

worin Ind für

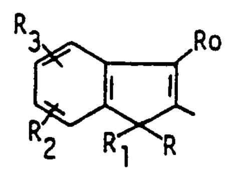

steht

und die Substituenten für Ind wie in Anspruch 1 definiert sind, und

worin $R_{13}$ ein einen Ester bildender Rest ist und $X_1$ $(CH_2)_n$ oder (E)—CH=CH— ist,

b) wenn X $(CH_2)_n$ oder (E)—CH=CH— ist und $R_{10}$ eine $C_1$—$C_3$--Alkylgruppe ist, durch Hydrolysieren einer Verbindung der Formel (XII)

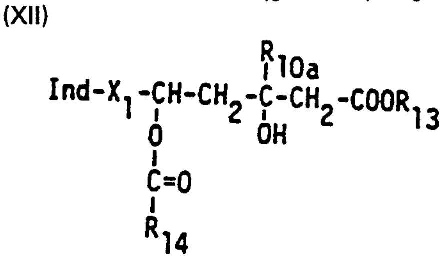

XII

worin $R_{10a}$ eine $C_1$—$C_3$-Alkylgruppe ist, $R_{14}$ Teil einer ester-bildenden Gruppe ist, und Ind, $X_1$ und $R_{13}$ wie oben definiert sind,

c) wenn X —CH=CH— oder —$CH_2$—CH=CH— ist und Z die Form eines δ-Lactons IIb in 4R,6S-Konfiguration hat, nämlich der Formel

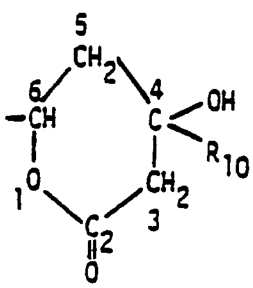

IIb

entspricht, worin $R_{10}$ wie im Anspruch 1 definiert ist, oder X $-CH_2CH_2$ oder $CH_2CH_2CH_2$ ist und IIb in 4R,6R-Konfiguration ist, Abspalten der Schutzgruppe einer Verbindung der Formel (XXXIX)

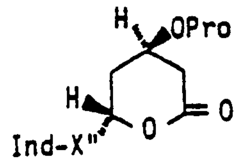

XXXIX

worin X'' $-CH_2CH_2$, $-CH_2CH_2CH_2$, $-CH=CH-$ oder $-CH_2CH=CH-$ ist und Pro eine Schutzgruppe ist, und Ind wie oben definiert ist,

d) wenn X $-(CH_2)_2-$, $-(CH_2)_3-$, $-(CH_2)_qCH=CH(CH_2)_q-$ ist, Abspalten der Schutzgruppe einer Verbindung der Formel (XXXII)

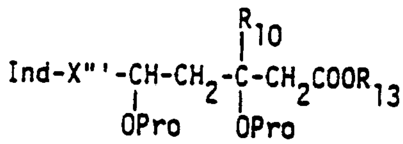

XXXII

worin X''' $-(CH_2)_2-$, $-(CH_2)_3-$ oder $-(CH_2)_q-CH=CH-(CH_2)_q$ ist, und Ind, q, $R_{10}$, $R_{13}$ und Pro wie oben definiert sind,

e) wenn Q

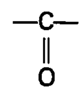

ist, Oxidieren der entsprechenden Verbindung der Formel (I), worin Q

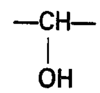

ist,

f) wenn

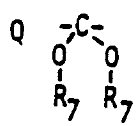

ist und II in Esterform ist, Ketalisieren der entsprechenden Verbindung der Formel (I), worin Q

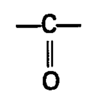

ist,

g) Hydrolysieren einer Verbindung der Formel (I) in Form eines Esters oder eines Lactons, oder

h) Verestern oder Bildung eines Lactons einer Verbindung der Formel (I) in freier Säureform, und wenn eine freie Carboxylgruppe vorhanden ist, Gewinnen der erhaltenen Verbindung in freier Säureform oder in Salzform.

14. Verbindung der Formel (IV)

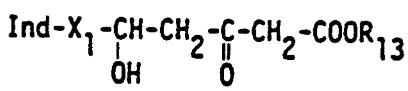

IV

worin $R_{13}$ ein esterbildender Rest ist, $X_1$ für $(CH_2)_n$ oder (E)$-CH=CH-$ steht und Ind wie im Anspruch 13 definiert ist, der Formel (XII)

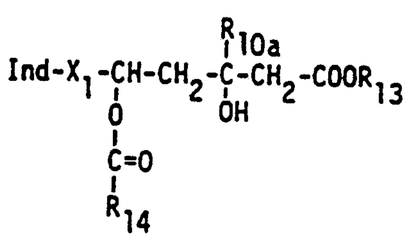

XII

worin $R_{10a}$ für $C_1$—$C_3$-Alkyl steht, $R_{14}$ Teil einer esterbildenden Gruppe ist und Ind, $X_1$ sowie $R_{13}$ wie oben definiert sind, der Formel (XXXII)

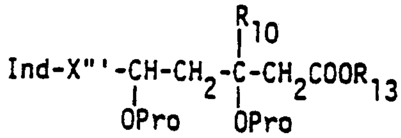

XXXII

in der X''' für $—(CH_2)_2—$, $—(CH_2)_3—$ oder $—(CH_2)_q—CH=CH—(CH_2)_q—$ steht, und Ind, q, $R_{10}$, $R_{13}$ sowie Pro wie oben definiert sind, oder der Formel (XXXIX)

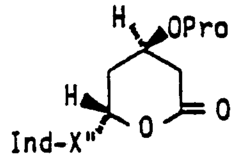

XXXIX

worin X'' für $—CH_2CH_2—$, $—CH_2CH_2CH_2—$, $—CH=CH—$ oder $—CH_2CH=CH—$ steht, Pro eine Schutzgruppe ist und Ind wie oben definiert ist.

**Patentansprüche für den Vertragsstaat: AT**

1. Verfahren zur Herstellung einer Verbindung der Formel (I)

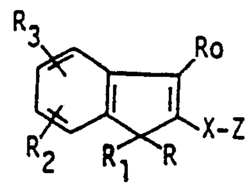

I

worin

R Wasserstoff oder eine primäre oder sekundäre $C_1$—$C_6$-Alkylgruppe,
$R_1$ eine primäre oder sekundäre $C_1$—$C_6$-Alkylgruppe oder
R und $R_1$ zusammen $(CH_2)_m$ oder $(Z)—CH_2—CH=CH—CH_2—$ sind, worin m 2, 3, 4, 5 oder 6 ist,
Ro eine $C_1$—$C_6$-Alkyl-, $C_3$—$C_7$-Cycloalkylgruppe oder ein Ring A

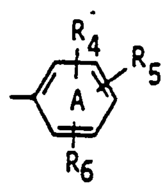

jeder $R_2$ und $R_4$ unabhängig Wasserstoff, eine $C_1$—$C_4$-Alkyl-, $C_1$—$C_4$-Alkoxy- (mit Ausnahme t-Butoxy), Trifluoromethyl-, Fluor-, Chlor-, Phenoxy- oder Benzyloxygruppe ist,
jeder $R_3$ und $R_5$ unabhängig Wasserstoff, eine $C_1$—$C_3$-Alkyl-, $C_1$—$C_3$-Alkoxy-, Trifluormethyl-, Fluor-, Chlor-, Phenoxy- oder Benzyloxygruppe ist.
$R_6$ Wasserstoff, eine $C_1$—$C_2$-Alkylgruppe, eine $C_1$—$C_2$-Alkoxygruppe, Fluor oder Chlor ist,
mit dem Vorbehalt, daß jeweils nur eine Trifluormethyl-, Phenoxy- oder Benzyloxygruppe an jedem der Phenyl- und Indenringe sein kann,
X $—(CH_2)_n—$ oder $—(CH_2)_qCH=CH(CH_2)_q—$ ist, worin n 1, 2 oder 3 ist und beide q 0 sind oder eines 0 und das andere 1 ist, und

Z is 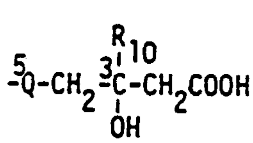 ist

II

worin

Q 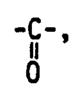, 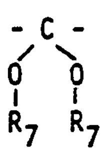 or 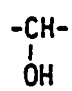

ist, worin jedes $R_7$ die gleiche primäre oder sekundäre $C_1$—$C_6$-Alkylgruppe ist oder zusammen —$(CH_2)_2$—, —$(CH_2)_3$— bilden, $R_{10}$ Wasserstoff oder eine $C_1$—$C_3$-Alkylgruppe ist, mit dem Vorbehalt, daß Q etwas anderes als

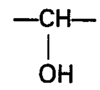

nur sein kann, wenn X —CH=CH— oder —$CH_2$—CH=CH— und/oder $R_{10}$ eine $C_1$—$C_3$-Alkylgruppe ist, in freier Säureform oder in Form eines Esters oder δ-Lactons davon oder in Salzform, gekennzeichnet,

a) wenn X $(CH_2)_n$ oder (E)—CH=CH— ist und $R_{10}$ Wasserstoff ist, durch Reduzieren einer Verbindung der Formel (IV)

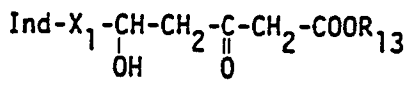

IV

worin Ind für

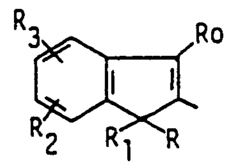

steht und die Substituenten für Ind wie oben definiert sind, und

worin $R_{13}$ ein einen Ester bildender Rest ist und $X_1$ $(CH_2)_n$ oder (E)—CH=CH— ist,

b) wenn X $(CH_2)_n$ oder (E)—CH=CH— ist und $R_{10}$ eine $C_1$—$C_3$-Alkylgruppe ist, durch Hydrolysieren einer Verbindung der Formel (XII)

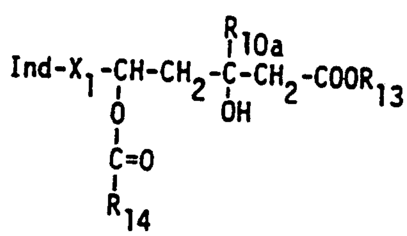

XII

worin $R_{10a}$ eine $C_1$—$C_3$-Alkylgruppe ist, $R_{14}$ Teil einer ester-bildenden Gruppe ist, und Ind, $X_1$ und $R_{13}$ wie oben definiert sind,

c) wenn X —CH=CH— oder —$CH_2$—CH=CH— ist und Z die Form eines δ-Lactons IIb in 4R,6S-Konfiguration hat, nämlich der Formel

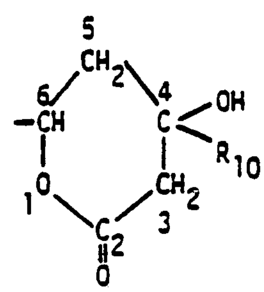

IIb

entspricht, worin $R_{10}$ wie oben definiert ist,

X —$CH_2CH_2$ oder $CH_2CH_2CH_2$ ist und IIb in 4R,6R-Konfiguration ist, Abspalten der Schutzgruppe einer Verbindung der Formel (XXXIX)

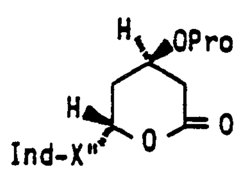

XXXIX

worin X'' $-CH_2CH_2$, $-CH_2CH_2CH_2$, $-CH=CH-$ oder $-CH_2CH=CH-$ ist und Pro eine Schutzgruppe ist, und Ind wie oben definiert ist,

d) wenn X $-(CH_2)_2-$, $-(CH_2)_3-$, $-(CH_2)_qCH=CH(CH_2)_q-$ ist, Abspalten der Schutzgruppe einer Verbindung der Formel (XXXII)

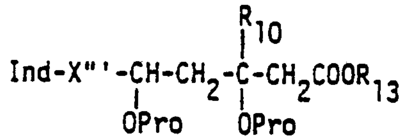

XXXII

worin X''' $-(CH_2)_2-$, $-(CH_2)_3-$ oder $-(CH_2)_q-CH=CH-(CH_2)_q-$ ist, und Ind, q, $R_{10}$, $R_{13}$ und Pro wie oben definiert sind,

e) wenn Q

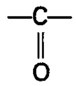

ist, Oxidieren der entsprechenden Verbindung der Formel (I), worin Q

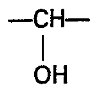

ist,

f) wenn

Q is 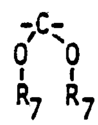

ist und II in Esterform ist, Ketalisieren der entsprechenden Verbindung der Formel (I), worin Q

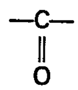

ist,

g) Hydrolysieren einer Verbindung der Formel (I) in Form eines Esters oder eines Lactons, oder

h) Verestern oder Bildung eines Lactons einer Verbindung der Formel (I) in freier Säureform, und wenn eine freie Carboxylgruppe vorhanden ist, Gewinnen der erhaltenen Verbindung in freier Säureform oder in Salzform.

2. Verfahren zur Herstellung einer Verbindung der Formel (I) gemäß Definition von Anspruch 1, dadurch gekennzeichnet, daß eine Verbindung der Formel (I) in Form eines Esters oder Lactons hydrolysiert oder eine Verbindung der Formel (I) in Form der freien Säure verestert oder lactonisiert wird und, falls eine freie Carboxylgruppe vorhanden ist, die erhaltene Verbindung in Form der freien Säure oder in Form eines Salzes gewonnen wird.

3. Verfahren nach Anspruch 1 oder 2 zur Herstellung einer Verbindung der Formel (I) gemäß Definition von Anspruch 1, ausgewählt aus *erythro*-(E)-3,5-Dihydroxy-7-[3'-(4''-fluorphenyl)-spiro[cyclopentan-1,1'(1H)-inden]-2'-yl]-hept-6-encarbonsäure und *erythro*-(E)-3,5-Dihydroxy-7-[3'-(3'',5''-dimethylphenyl)-spiro[cyclopentan-1,1'(1H)-inden]-2'-yl]-hept-6-encarbonsäure in Form der freien Säure oder in Form eines Salzes.

4. Verfahren nach Anspruch 1 oder 2 zur Herstellung einer Verbindung gemäß Anspruch 3 in Form eines Natriumsalzes.

5. Verfahren nach Anspruch 1 oder 2 zur Herstellung von Natrium-*erythro*-(E)-3,5-dihydroxy-7-[3'-(4''-fluorphenyl)-spiro[cyclopentan-1,1'(1H)-inden]-2'-yl]-hept-6-enoat in Form der freien Säure oder in Form eines Salzes.

6. Verfahren nach Anspruch 1 oder 2 zur Herstellung des (3R,5S)-Enantiomers der Verbindung von Anspruch 5.

7. Verfahren zur Herstellung einer pharmazeutischen Zusammensetzung, dadurch gekennzeichnet, daß eine Verbindung der Formel (I) gemäß Definition von Anspruch 1 in Form der freien Säure oder in Form eines pharmazeutisch annehmbaren Esters oder δ-Lactons hiervon oder in Form eines pharmazeutisch annehmbaren Salzes mit einem pharmazeutisch annehmbaren Träger oder Verdünnungsmittel vermischt wird.

**Revendications pour les Etats contractants: BE CH DE FR GB IT LI LU NL SE**

1. Une composé de formule I

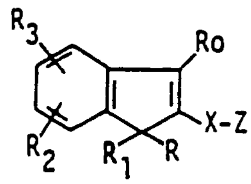

I

dans laquelle

R signifie l'hydrogène ou alkyle primaire ou secondaire en $C_1$—$C_6$,

$R_1$ signifie alkyhle primaire ou secondaire en $C_1$—$C_6$, ou bien

R et $R_1$ signifient ensemble $(CH_2)_m$ ou (Z)—$CH_2$—CH=CH—$CH_2$— où m signifie 2, 3, 4, 5 ou 6,

Ro signifie alkyle en $C_1$—$C_6$, cycloalkyle en $C_3$—$C_7$ ou un cycle A

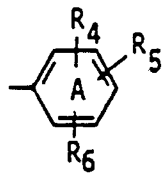

chaque $R_2$ et $R_4$ signifie indépendamment l'hydrogène, alkyle en $C_1$—$C_4$, alcoxy en $C_1$—$C_4$ (sauf tert.-butoxy), trifluorométhyle, fluoro, chloro, phénoxy ou benzyloxy,

chauque $R_3$ et $R_5$ signifie indépendamment l'hydrogène, alkyle en $C_1$—$C_3$, alcoxy en $C_1$—$C_3$, trifluorométhyle, fluoro, chloro, phénoxy ou benzyloxy,

$R_6$ signifie l'hydrogène, alkyle en $C_1$—$C_2$, alcoxy en $C_1$—$C_2$, fluoro ou chloro,

un seul groupe trifluorométhyle, phénoxy ou benzyloxy pouvant se trouver sur chacun des cycles phényle et indène,

X signifie —$(CH_2)_n$— ou —$(CH_2)_q$CH=CH$(CH_2)_q$— où n signifie 1, 2 ou 3 et les deux symboles q signifient 0 ou l'un signifie 0 et l'autre signifie 1,

et

Z signifie 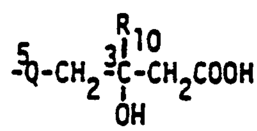 II

où

Q signifie 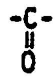, 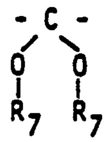 ou 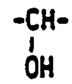

où chaque $R_7$ signifie le même alkyle primaire ou secondaire en $C_1$—$C_6$ ou signifient ensemble —$(CH_2)_2$—, —$(CH_2)_3$—,

$R_{10}$ signifie l'hydrogène ou alkyle en $C_1$—$C_3$, Q pouvant avoir une signification autre que —CH(OH)— seulement lorsque X signifie —CH=CH— ou —$CH_2$—CH=CH— et/ou $R_{10}$ signifie alkyle en $C_1$—$C_3$, comme cela convient sous forme d'acide libre ou sous forme d'ester ou de δ-lactone de composé ou sous forme de sel.

2. Un composé selon la revendication 1, dans lequel

Ro signifie un cycle A

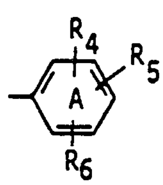

R signifie l'hydrogène ou alkyle primaire ou secondaire en $C_1$—$C_6$ ne contenant pas d'atome de carbone asymétrique, et

$R_1$ signifie alkyle primaire ou secondaire en $C_1$—$C_6$ ne contenant pas d'atome de carbone asymétrique, ou bien

R et $R_1$ pris ensemble signifient —$(CH_2)_m$— ou (Z)—$CH_2$—CH=CH—$CH_2$—, où m signifie 2, 3, 4, 5 ou 6,

$R_2$ signifie l'hydrogène, alkyle en $C_1$—$C_3$, n-butyle, iso-butyle, tert.-butyle, alcoxy en $C_1$—$C_3$, n-butoxy, iso-butoxy, trifluorométhyle, fluoro, chloro, phénoxy ou benzyloxy,

$R_3$ signifie l'hydrogène, alkyle en $C_1$—$C_3$, alcoxy en $C_1$—$C_3$, trifluorométhyle, fluoro, chloro, phénoxy ou benzyloxy,

un seul des symboles $R_2$ et $R_3$ pouvant signifier un groupe trifluorométhyle, un seul des symboles $R_2$ et $R_3$ pouvant signifier un groupe phénoxy, et un seul des symboles $R_2$ et $R_3$ pouvant signifier un groupe benzyloxy,

$R_4$ signifie l'hydrogène, alkyle en $C_1$—$C_3$, n-butyle, iso-butyle, tert.-butyle, alcoxy en $C_1$—$C_3$, n-butoxy, iso-butoxy, trifluorométhyle, fluoro, chloro, phénoxy ou benzyloxy,

$R_5$ signifie l'hydrogène, alkyle en $C_1$—$C_3$, alcoxy en $C_1$—$C_3$, trifluorométhyle, fluoro, chloro, phénoxy ou benzyloxy,

$R_6$ signifie l'hydrogène, alkyle en $C_1$—$C_2$, alcoxy en $C_1$—$C_2$, fluoro ou chloro,

un seul des symboles $R_4$ et $R_5$ pouvant signifier un groupe trifluorométhyle, un seul des symboles $R_4$ et $R_5$ pouvant signifier un groupe phénoxy et un seul des symboles $R_4$ et $R_5$ pouvant signifier un groupe benzyloxy,

X signifie —$(CH_2)_n$— ou (E)—CH=CH—, où n signifie 1, 2 ou 3, et

Z signifie 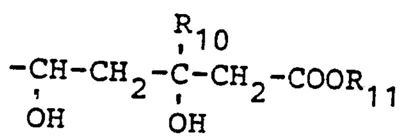 II(a) ou 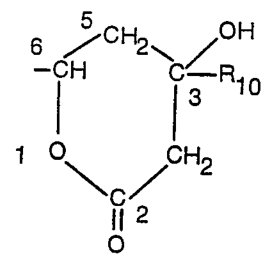 II(b)

où

$R_{10}$ signifie l'hydrogène ou alkyle en $C_1$—$C_3$, et

$R_{11}$ signifie l'hydrogène, $R_{12}$ ou M,

$R_{12}$ signifiant un groupe ester physiologiquement acceptables et physiologiquement hydrolysable, et

M signifiant un cation pharmaceutiquement acceptable.

3. Un composé selon la revendication 1, dans lequel Ro signifie 4-fluorophényle.

4. Une composé choisi parmi l'acide érythro-(E)-3,5-dihydroxy-7-[3'-(4''-fluorophényl)-spiro[cyclopentane-1,1'(1H)-indène]-2'-yl]-hept-6-énoïque et l'acide érythro-(E)-3,5-dihydroxy-7-[3'-(3'',5''-diméthylphényl)-spiro[cyclopentane-1,1'(1H)-indène]-2'-yl]-hept-6-énoïque, sous forme d'acide libre ou de sel.

5. Un composé selon la revendication 4, sous forme de sel de sodium.

6. Un composé selon la revendication 1, qui est l'érythro-(E)-3,5-dihydroxy-7-[3'-(4''-fluorophényl)-spiro[cyclopentane-1,1'(1H)-indène]-2'-yl]-hept-6-énoate, sous forme d'acide libre ou de sel.

7. Un composé selon la revendication 1, qui est l'énantiomère (3R,5S) d'un composé selon la revendication 6, sous forme d'acide libre ou de sel.

8. Un composé selon la revendication 6 ou 7, sous forme de sel de sodium.

9. Une composition pharmaceutique comprenant un composé selon la revendication 1, comme cela convient sous formé d'acide libre ou sous forme d'un ester physiologiquement hydrolysable et physiologiquement acceptable ou d'une lactone de ce composé ou sous forme d'un sel pharmaceutiquement acceptable, ensemble avec un diluant ou véhicule pharmaceutiquement acceptable.

10. Un composé selon la revendication 1, comme cel a convient sous forme d'acide libre ou sous forme d'un ester physiologiquement hydrolysable et physiologiquement acceptable ou d'une lactone de ce composé ou sous forme d'un sel pharmaceutiquement acceptable, pour l'utilisation comme médicament.

11. Un composé selon la revendication 1, comme cela convient sous forme d'acide libre ou sous forme d'un ester physiologiquement hydrolysable et physiologiquement acceptable ou d'une lactone de ce composé ou sous forme d'un sel pharmaceutiquement acceptable, pour l'utilisation pour inhiber la biosynthèse du cholestérol ou pour le traitement de l'athérosclérose.

12. Un procédé de préparation d'un composé selon la revendication 1, comprenant l'hydrolyse d'un composé de formule I sous forme d'ester ou de lactone ou l'estérification ou la lactonisation d'un composé de formule I sous forme d'acide libre, et lorsqu'un groupe carboxy libre est présent, la récupération du composé obtenu sous forme d'acide libre ou sous forme d'une sel.

13. Un procédé de préparation d'un composé selon la revendication 1, qui comprend

a) lorsque X signifie $(CH_2)_n$ ou (E)—CH=CH— et $R_{10}$ signifie l'hydrogène, la réduction d'un composé de formule IV

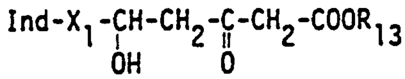

IV

dans laquelle Ind signifie

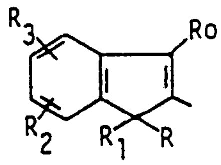

et les substituants de Ind sont tels que définis à la revendication 1, et dans laquelle $R_{13}$ est un radical formant un ester et $X_1$ signifie $(CH_2)_n$ ou (E)—CH=CH—,

b) lorsque X signifie $(CH_2)_n$ ou (E)—CH=CH— et $R_{10}$ signifie alkyle en $C_1$—$C_3$, l'hydrolyse d'un composé de formule XII

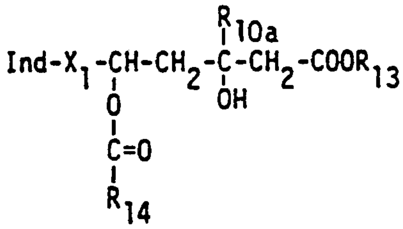

XII

dans laquelle $R_{10a}$ signifie alkyle en $C_1$—$C_3$, $R_{14}$ est la partie d'un groupe formant un ester et Ind, $X_1$ et $R_{13}$ sont tels que définis plus haut,

c) lorsque X signifie —CH=CH— ou —$CH_2$—CH=CH— et le groupe Z est sous forme d'une δ-lactone IIb ayant la configuration 4R,6S,

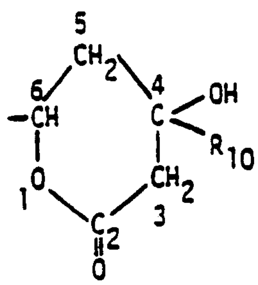

IIb

où $R_{10}$ est tel que défini à la revendication 1, ou bien lorsque

X signifie —$CH_2CH_2$ ou $CH_2CH_2CH_2$ et IIb a la configuration 4R,6R, la déprotection d'un composé de formule XXXIX

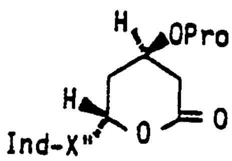

XXXIX

où X'' signifie —$CH_2CH_2$, —$CH_2CH_2CH_2$, —CH=CH— ou —$CH_2$CH=CH— et Pro signifie un groupe protecteur et Ind est tel que défini plus haut,

d) lorsque X signifie —$(CH_2)_2$—, —$(CH_2)_3$—, —$(CH_2)_q$CH=CH$(CH_2)_q$—, la déprotection d'une composé de formule XXXII

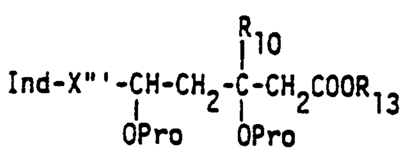

XXXII

où X''' signifie —$(CH_2)_2$—, —$(CH_2)_3$— ou —$(CH_2)_q$—CH=CH—$(CH_2)_q$, et Ind, q, $R_{10}$, $R_{13}$ et Pro sont tels que définis plus haut,

e) lorsque Q signifie —CO—, l'oxydatioon du composé correspondant de formule I où Q signifie —CH(OH)—,

f) lorsque

Q signifie 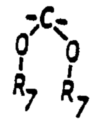

et II est sous forme d'ester, la cétalisation du composé correspondant de formule I où Q signifie —CO—,

g) l'hydrolyse d'un composé de formule I sous forme d'ester ou de lactone, ou

h) l'esterification ou la lactonisation d'un composé de formule I sous forme d'acide libre,

et, lorsqu'un groupe carboxy libre est présent, la récupération du composé obtenu sous forme d'acide libre ou sous forme d'un sel.

14. Un composé de formule IV

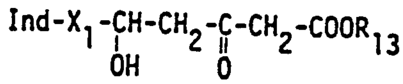

IV

dans laquelle $R_{13}$ est un radical formant un ester, $X_1$ signifie $(CH_2)_n$ ou (E)—CH=CH— et Ind est tel que défini à la revendication 13;

de formule XII

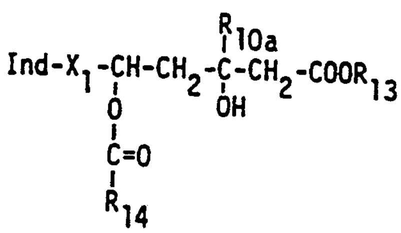

XII

dans laquelle $R_{10a}$ signifie alkyle en $C_1$—$C_3$, $R_{14}$ est la partie d'un groupe formant un ester et Ind, $X_1$ et $R_{13}$ sont tels que définis plus haut;

de formule XXXII

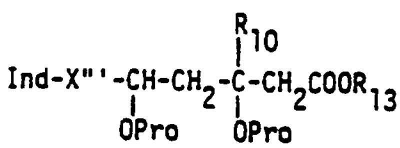

XXXII

dans laquelle X''' signifie $—(CH_2)_2—$, $—(CH_2)_3—$ ou $—(CH_2)_q—CH=CH—(CH_2)_q—$ et Ind, q, $R_{10}$, $R_{13}$ et Pro sont tels que définis plus haut;

ou de formule XXXIX

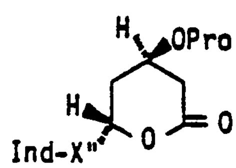

XXXIX

dans laquelle X'' signifie $—CH_2CH_2—$, $—CH_2CH_2CH_2—$, —CH=CH— ou $—CH_2CH=CH—$, Pro siginifie un groupe protecteur et Ind est tel que défini plus haut.

**Revendications pour l'Etat contractant: ET**

1. Un procédé de préparation d'un composé de formule I

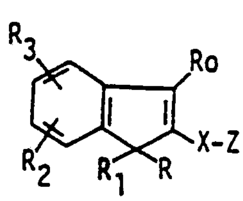

I

dans laquelle

R signifie l'hydrogène ou alkyle primaire ou secondaire en $C_1$—$C_6$,

$R_1$ signifie alkyle primaire ou secondaire en $C_1$—$C_6$, ou bien

R et $R_1$ signifient ensemble $(CH_2)_m$ ou (Z)—$CH_2$—CH=CH—$CH_2$— où m signifie 2, 3, 4, 5 ou 6,

Ro signifie alkyle en $C_1$—$C_6$, cycloalkyle en $C_3$—$C_7$ ou un cycle A

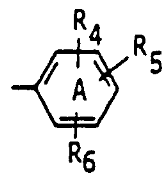

chaque $R_2$ et $R_4$ signifie indépendamment l'hydrogène ou alkyle en $C_1$—$C_4$, alcoxy en $C_1$—$C_4$ (sauf tert.-butoxy), trifluorométhyle, fluoro, chloro, phénoxy ou benzyloxy,

chaque $R_3$ et $R_5$ signifie indépendamment l'hydrogène ou alkyle en $C_1$—$C_3$, alcoxy en $C_1$—$C_3$, trifluorométhyle, fluoro, chloro, phénoxy ou benzyloxy,

$R_6$ signifie l'hydrogène, alkyle en $C_1$—$C_2$, alcoxy en $C_1$—$C_2$, fluoro ou chloro,

un seul groupe trifluorométhyle, phénoxy ou benzyloxy pouvant se trouver sur chacun des cycles phényle et indène,

X signifie —$(CH_2)_n$— ou —$(CH_2)_q$CH=CH$(CH_2)_q$— où n signifie 1, 2 ou 3 et les deux symboles q signifient 0 ou l'un signifie 0 et l'autre signifie 1,

et

Z signifie 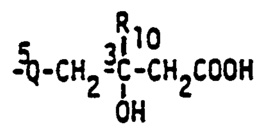 II

où

Q signifie 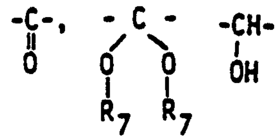

où chaque $R_7$ signifie le même alkyle primaire ou secondaire en $C_1$—$C_6$ ou signifient ensemble —$(CH_2)_2$—, —$(CH_2)_3$—,

$R_{10}$ signifie l'hydrogène ou alkyle en $C_1$—$C_3$, Q pouvant avoir une signification autre que —CH(OH)— seulement lorsque X signifie —CH=CH— ou —$CH_2$—CH=CH— et/ou $R_{10}$ signifie alkyle en $C_1$—$C_3$, comme cela convient sous forme d'acide libre ou sous forme d'ester ou de δ-lactone de composé ou sous forme de sel,

qui comprend

a) lorsque X signifie $(CH_2)_n$ ou (E)—CH=CH— et $R_{10}$ signifie l'hydrogène, la réduction d'un composé de formule IV

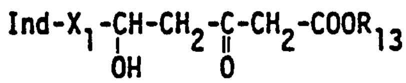 IV

dans laquelle Ind signifie

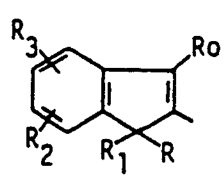

et les substituants de Ind sont tels que définis à plus haut, et dans laquelle $R_{13}$ est un radical formant un ester et $X_1$ signifie $(CH_2)_n$ ou (E)—CH=CH—,

b) lorsque X signifie $(CH_2)_n$ ou (E)—CH=CH— et $R_{10}$ signifie alkyle en $C_1$—$C_3$, l'hydrolyse d'un composé de formule XII

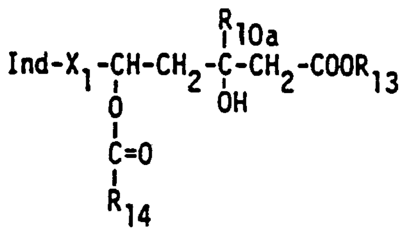

XII

dans laquelle $R_{10a}$ signifie alkyle en $C_1$—$C_3$, $R_{14}$ est la partie d'un groupe formant un ester et Ind, $X_1$ et $R_{13}$ sont tels que définis plus haut,

c) lorsque X signifie —CH=CH— ou —$CH_2$—CH=CH— et le groupe Z est sous forme d'une δ-lactone de formule IIb ayant la configuration 4R,6S,

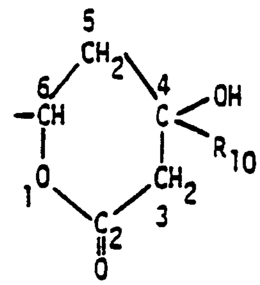

IIb

où $R_{10}$ est tel que défini à la revendication 1, ou bien lorsque

X signifie —$CH_2CH_2$ ou $CH_2CH_2CH_2$ et IIb a la configuration 4R,6R, la déprotection d'un composé de formule XXXIX

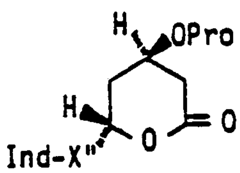

XXXIX

où X'' signifie —$CH_2CH_2$, —$CH_2CH_2CH_2$, —CH=CH— ou —$CH_2$CH=CH— et Pro signifie un groupe protecteur et Ind est tel que défini plus haut,

d) lorsque X signifie —$(CH_2)_2$—, —$(CH_2)_3$—, —$(CH_2)_q$CH=CH$(CH_2)_q$—, la déprotection d'une composé de formule XXXII

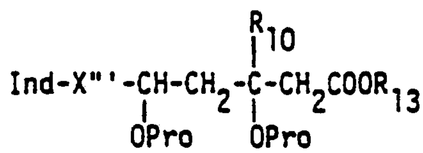

XXXII

où X''' signifie —$(CH_2)_2$—, —$(CH_2)_3$— ou —$(CH_2)_q$—CH=CH—$(CH_2)_q$, et Ind, q, $R_{10}$, $R_{13}$ et Pro sont tels que définis plus haut,

e) lorsque Q signifie —CO—, l'oxydation du composé correspondant de formule I où Q signifie —CH(OH)—,

f) lorsque Q signifie

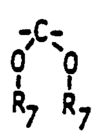

et II est sous forme d'ester, la cétalisation du composé correspondant de formule I où Q signifie —CO—,

g) l'hydrolyse d'un composé de formule I sous forme d'ester ou de lactone ou

h) l'esterification ou la lactonisation d'un composé de formule I sous forme d'acide libre,

et, lorsqu'un groupe carboxy libre est présent, la récupération du composé obtenu sous forme d'acide libre ou sous forme d'un sel.

2. Un procédé de préparation d'un composé de formule I tel que défini à la revendication 1, qui comprend l'hydrolyse d'un composé de formule I sous forme d'ester ou de lactone ou l'estérification ou la lactonisation d'un composé de formule I sous forme d'acide libre et, lorsqu'un groupe carboxy libre est présent, la récupération du composé obtenu sous forme d'acide libre ou sous forme d'un sel.

3. Un procédé selon la revendication 1 ou 2, pour la préparation d'un composé de formule I tel que défini à la revendication 1, choisi parmi l'acide érythro-(E)-3,5-dihydroxy-7-[3'-(4''-fluorophényl)-spiro[cyclopentane-1,1'(1H)-indène]-2'-yl]-hept-6-énoïque et l'acide érythro-(E)-3,5-dihydroxy-7-[3'-(3'',5''-diméthylphényl)-spiro[cyclopentane-1,1'(1H)-indène]-2'-yl]-hept-6-énoïque, sous forme d'acide libre ou de sel.

4. Un procédé selon la revendication 1 ou 2, pour la préparation d'un composé selon la revendication 3 sous forme de sel de sodium.

5. Une procédé selon la revendication 1 ou 2, pour la préparation de érythro-(E)-3,5-dihydroxy-7-[3'-(4''-fluorophényl)-spiro[cyclopentane-1,1'(1H)-indène]-2'-yl]-hept-6-énoate de sodium, sous forme d'acide libre ou de sel.

6. Un procédé selon la revendication 1 ou 2, pour la préparation de l'énantiomère (3R,5S) du composé selon la revendication 5.

7. Un procédé de préparation d'une composition pharmaceutique, qui comprend le mélange d'un composé de formule I tel que défini à la revendication 1, comme cela convient sous forme d'acide libre ou sous forme d'un ester ou d'une δ-lactone pharmaceutiquement acceptable de ce composé ou sous forme d'un sel pharmaceutiquement acceptable, avec un véhicule ou diluant pharmaceutiquement acceptable.